# EUROPEAN PATENT SPECIFICATION

(11) **EP 3 503 901 B1**
(45) Date of publication and mention of the grant of the patent: **28.09.2022**
(21) Application number: 17847396.3
(22) Date of filing: 29.08.2017
(51) Int. Cl.: C12N 5/071, C12N 5/079, C12N 5/0793, C12N 5/0797, A61K 35/30

(54) **METHODS AND COMPOSITIONS FOR SPINAL CORD CELLS**
VERFAHREN UND ZUSAMMENSETZUNGEN FÜR RÜCKENMARKSZELLEN
PROCÉDÉS ET COMPOSITIONS POUR CELLULES DE MOELLE ÉPINIÈRE

(30) Priority: 29.08.2016 US 201662380780 P; 19.10.2016 WO PCT/US2016/057724; 15.11.2016 US 201615352289
(43) Date of publication of application: 03.07.2019
(73) Proprietor: Cedars-Sinai Medical Center, Los Angeles, CA 90048 (US)
(72) Inventor: VATINE, Gad, Los Angeles California 90026 (US); SANCES, Sam, Santa Monica California 90405 (US); SVENDSEN, Clive, Pacifica Palisades California 90272 (US); SAREEN, Dhruv, Porter Ranch California 91326 (US); KERL, Alexis J., Northridge CA 91324 (US)
(74) Representative: Hutter, Anton
(86) International application number: PCT/US2017/049193
(87) International publication number: WO 2018/044934

(56) References cited:
- EP-A1- 3 031 908
- WO-A1-2015/163823
- WO-A1-2015/181253
- WO-A2-2010/108005
- US-A1- 2013 224 857
- US-A1- 2016 152 950
- DHRUV SAREEN ET AL: "Human induced pluripotent stem cells are a novel source of neural progenitor cells (iNPCs) that migrate and integrate in the rodent spinal cord : Human neural progenitor cells", JOURNAL OF COMPARATIVE NEUROLOGY., vol. 522, no. 12, 12 April 2014 (2014-04-12), pages 2707-2728, XP055384684, US ISSN: 0021-9967, DOI: 10.1002/cne.23578
- DAISUKE SHIMOJO ET AL: "Rapid, efficient, and simple motor neuron differentiation from human pluripotent stem cells", MOLECULAR BRAIN, vol. 8, no. 1, 1 December 2015 (2015-12-01), XP055657428, DOI: 10.1186/s13041-015-0172-4
- KUN QIAN ET AL: "A Simple and Efficient System for Regulating Gene Expression in Human Pluripotent Stem Cells and Derivatives : Inducible Transgene Expression in hPSCs and Derivatives", STEM CELLS (MIAMISBURG), vol. 32, no. 5, 17 April 2014 (2014-04-17) , pages 1230-1238, XP055441569, ISSN: 1066-5099, DOI: 10.1002/stem.1653
- HU WENTAO ET AL: "Derivation, Expansion, and Motor Neuron Differentiation of Human-Induced Pluripotent Stem Cells with Non-Integrating Episomal Vectors and a Defined Xenogeneic-free Culture System", MOLECULAR NEUROBIOLOGY, HUMANA PRESS, US, vol. 53, no. 3, 10 February 2015 (2015-02-10), pages 1589-1600, XP036236338, ISSN: 0893-7648, DOI: 10.1007/S12035-014-9084-Z [retrieved on 2015-02-10]
- SAREEN, D ET AL.: 'Human neural progenitor cells generated from induced pluripotent stem cells can survive, migrate, and integrate in the rodent spinal cord' JOURNAL OF COMPARATIVE NEUROLOGY vol. 522, no. 12, 12 April 2014, pages 2707 - 2728, XP055384684

## Description

### FIELD OF THE INVENTION

The invention relates to culturing neuronal lineage cells, particularly spinal cord progenitors, together with endothelial cells in a fluidic device under conditions whereby the cells mimic the structure and function of developmental structures.

### BACKGROUND OF THE INVENTION

Human stem cell derived models of neurodegenerative diseases are challenged by immaturity *in vitro.* Developing neurons *in vivo* interact with multiple non-neuronal cell types as they mature. Microphysiological systems (MPS), also known as Organ-Chips, possess novel microvolume culture geometries that also enable co-culture of distinct cell types relevant for organ function. Brain Microvascular endothelial cells (BMECs) share many common signaling pathways with neurons early in development, however their contribution to human neuronal maturation is largely unknown. There is a great need in the art to develop differentiation systems that faithfully mirror *in vivo* developmental structures and processes.

Described here are systems and methods for deriving both spinal motor neurons and brain microvascular endothelial cells from induced pluripotent stem cells using distinct methods and combining them in a chip format. Neurons cultured alone in chip microvolume displayed increased calcium transient function and chip-specific gene expression compared to 96 well plates. BMECs seeded into a distinct channel in the chip directly contacted developing neural cultures and this interaction further enhanced neural function and elicited vascular-neural interaction and niche genes. Transcriptomic comparison to fetal and adult spinal cord tissue revealed enhanced *in vivo*-like signatures arising from the chip co-cultures. Development of novel media formulations further allow for improved readout of differentiation process, by eliminating additives that otherwise confound differentiation processes and resulting phenotypes.

EP3031908 teaches a method for producing dopaminergic neurons from induced pluripotent stem cells. Sareen et al., Journal of Comparative Neurology, vol. 522, no. 12, 12 April 2014, pages 2707-2728, discloses neural progenitor cell generation from iPSC-derived EZ spheres. Shimojo et al., Molecular Brain, vol. 8, no. 1, 1 December 2015, pages 1-15, teaches the derivation of motor neurons from hiPSCs comprising treatment of iPSCs with CHIR99021, LDN193189 and SB431542. Qian et al., Stem Cells, vol. 32, no. 5, 17 April 2014, pages 1230-1238, teaches neural differentiation of hPSCs. Wentao et al., Molecular Neurobiology, vol. 53, no. 3, 10 February 2015, pages 1589-1600 describes motor neuron induction from iPSCs. WO 2015/163823 discloses a medium selected from B-27 supplement.

### SUMMARY OF THE INVENTION

The invention is as defined in the claims.

In a first aspect of the invention, there is provided a method of generating spinal neural progenitor cells (spNPCs) comprising: providing induced pluripotent stem cells (iPSCs); differentiating iPSCs into neural ectodermal cells by culturing in neural induction media comprising LDN193189, SB431542, and CHIR99021 for about 5-7 days; and dissociating neural ectodermal cells, replating on a cell culture substrate and further culturing the replated neural ectodermal cells in differentiation media for about 5-7 days to generate spNPCs, wherein the differentiation media comprises retinoic acid and sonic hedgehog agonist (SAG).

In a second aspect of the invention, there is provided a cell culture supplement, comprising: Albumin, Bovine Serum, Sodium Bicarbonate, L-Ascorbic Acid, Putrescine, D(+)-Galactose, Holo-transferrin, Catalase, L-Carnitine, Glutathione (reduced), Sodium Selenite, Ethanolamine, and T3 (triiodo-L-thyronine), Corticosterone, Linoleic Acid, Linolenic Acid, Lipoic Acid (thioctic acid), Progesterone, Retinol Acetate, a-Tocopherol (vitamin E), and a-Tocopherol acetate.

In a third aspect of the invention, there is provided a cell culture media, comprising the cell culture supplement of the second aspect, and a base media, optionally wherein the base media comprises DMEM/F12.

In a fourth aspect of the invention, there is provided a method of generating spinal neural progenitor cells (spNPCs) comprising: providing induced pluripotent stem cells (iPSCs); differentiating iPSCs into neural ectodermal cells by culturing in neural induction media comprising LDN193189, SB431542, and CHIR99021 for about 6 days; and dissociating neural ectodermal cells, replating on a cell culture substrate and further culturing the replated neural ectodermal cells in differentiation media for about 6 days to generate spNPCs, wherein the neural induction media, differentiation media, or both, comprise a supplement comprising: one or more first additives selected from the group consisting of: Albumin, Bovine Serum, Sodium Bicarbonate, L-Ascorbic Acid, Putrescine, D(+)-Galactose, Holo-transferrin, Catalase, L-Carnitine, Glutathione (reduced), Sodium Selenite, Ethanolamine, and T3 (triiodo-L-thyronine); and one or more second additives selected from the group consisting of: Corticosterone, Linoleic Acid, Linolenic Acid, Lipoic Acid (thioctic acid), Progesterone, Retinol Acetate, a-Tocopherol (vitamin E), and a-Tocopherol acetate, and wherein the differentiation media comprises retinoic acid and sonic hedgehog agonist (SAG).

In a fifth aspect of the invention, there is provided a quantity of spNPCs made by the method of the fourth aspect, wherein the spNPCs express NKX6.1, optionally TUBB3, and at least one of ISL1 and SMI32.

In a sixth aspect of the invention, there is provided a cryopreserved solution of spNPCs made by the method of the first or fourth aspect, wherein the spNPCs express NKX6.1, optionally TUBB3, and at least one of ISL1 and SMI32.

Described herein as an example, and not forming part of the invention, is a method of generating spinal neural progenitor cells (spNPCs) including providing induced pluripotent stem cells (iPSCs), differentiating iPSCs into neural ectodermal cells by culturing in neural induction media, and dissociating neural ectodermal cells, replating on a cell culture substrate and further culturing the replated neural ectodermal cells in differentiation media to generate spNPCs. In other examples, the iPSCs are cultured in neural induction media for a period of about 5-7 days. In other examples, the replated neural ectodermal cells are cultured in differentiation media for a period of about 5-7 days. In other examples, the neural induction media comprises one or more of: LDN193189, SB431542, and CHIR99021. In other examples, the cell culture substrate is matrigel. In other examples, the differentiation media comprises one or more of: ascorbic acid, retinoic acid, SAG. In other examples, the method further includes freezing the spNPCs.

Also described herein as an example, and not forming part of the invention, is a cryopreserved solution of spNPCs made by is a method of generating spinal neural progenitor cells (spNPCs) including providing induced pluripotent stem cells (iPSCs), differentiating iPSCs into neural ectodermal cells by culturing in neural induction media, and dissociating neural ectodermal cells, replating on a cell culture substrate and further culturing the replated neural ectodermal cells in differentiation media to generate spNPCs.

Described herein as an example, and not forming part of the invention, is a cell culture supplement, including one or more first additives selected from the group consisting of: Albumin, Bovine Serum, Sodium Bicarbonate, L-Ascorbic Acid, Putrescine, D(+)-Galactose, Holo-transferrin, Catalase, L-Carnitine, Glutathione (reduced), Sodium Selenite, Ethanolamine, and T3 (triiodo-L-thyronine), and one or more second additives selected from the group consisting of: Corticosterone, Linoleic Acid, Linolenic Acid, Lipoic Acid (thioctic acid), Progesterone, Retinol Acetate, a-Tocopherol (vitamin E), and a-Tocopherol acetate. Further described herein is a culture media including the cell culture supplement and a base media. In other examples, the base media comprises DMEM/F12.

Also described herein as an example, and not forming part of the invention, is a method of generating spinal neural progenitor cells (spNPCs) including providing induced pluripotent stem cells (iPSCs), differentiating iPSCs into neural ectodermal cells by culturing in neural induction media for about 6 days, and dissociating neural ectodermal cells, replating on a cell culture substrate and further culturing the replated neural ectodermal cells in differentiation media for about 6 days to generate spNPCs. In other examples, the neural induction media, differentiation media, or both, comprise a supplement including: one or more first additives selected from the group consisting of: Albumin, Bovine Serum, Sodium Bicarbonate, L-Ascorbic Acid, Putrescine, D(+)-Galactose, Holo-transferrin, Catalase, L-Carnitine, Glutathione (reduced), Sodium Selenite, Ethanolamine, and T3 (triiodo-L-thyronine), and one or more second additives selected from the group consisting of: Corticosterone, Linoleic Acid, Linolenic Acid, Lipoic Acid (thioctic acid), Progesterone, Retinol Acetate, a-Tocopherol (vitamin E), and a-Tocopherol acetate. In other examples, the neural induction media comprises one or more of: LDN193189, SB431542, and CHIR99021. In other examples, the cell culture substrate is matrigel. In other examples, the differentiation media comprises one or more of: ascorbic acid, retinoic acid, SAG. Also described herein is a quantity of spNPCs, made by the aforementioned method. In other examples, the method further includes freezing the spNPCs. Further described herein is a cryopreserved solution of spNPCs made by the aforementioned method.

### BRIEF DESCRIPTION OF THE FIGURES

**Figure 1****. Depiction of protocol for cellular differentiation, including use of SLDM media.** Sareen lab differentiation media (SLDM) is a new supplement method made in the Inventors' lab, while IMDM/F12 is the traditional method of making the spinal cord cells. Whereas differentiating iPSCs for 6 days using IMDM/F12 method could lead to sample loss due to cell death after dissociating and re-seeding on this day, SLDM improves adherence and viability of differentiating cells.
**Figure 2****. Comparative study of media including IMDM vs SLDM. Day 6, 5x magnification.** At day 6, neuroepithelia are formed.
**Figure 3****. Comparative study of media including IMDM vs SLDM. Day 6, 10x magnification.** Seen here are cells cultured in IMDM possessing folds and curves that are indicative of inability to survive dissociation and re-seeding.
**Figure 4****. Comparative study of media including IMDM vs SLDM. Day 6, 20x magnification.**
**Figure 5****. Comparative study of media including IMDM vs SLDM. Day 12, 5x magnification.** At day 12, motor neuron precursors are formed. Here, cells in SLDM method look very uniform at this day 12 stage.
**Figure 6****. Comparative study of media including IMDM vs SLDM. Day 12, 10x magnification.**
**Figure 7****. Comparative study of media including IMDM vs SLDM. Day 12, 20x magnification.**
**Figure 8****. Comparative study of media including IMDM vs SLDM. Day 12,** 5x **magnification.**
**Figure 9****. Comparative study of media including IMDM vs SLDM. Day 12, 10x magnification.**
**Figure 10****. Comparative study of media including IMDM vs SLDM. Day 12, 20x magnification.**
**Figure 11****. Comparative study of media including IMDM vs SLDM. Day 18, 5x magnification.**
**Figure 12****. Comparative study of media including IMDM vs SLDM. Day 18, 10x magnification.**
**Figure 13****. Comparative study of media including IMDM vs SLDM. Day 18, 20x magnification.** Cells/ neurons on day 18 in IMDM/F12 are in tight clumps, preventing effective imaging analysis.
**Figure 14****. Comparative study of media including IMDM vs SLDM. Day 18, 5x magnification.**
**Figure 15****. Comparative study of media including IMDM vs SLDM. Day 18, 10x magnification.**
**Figure 16****. Comparative study of media including IMDM vs SLDM. Day 18, 20x magnification.**
**Figure 17****. Comparative study of media including IMDM vs SLDM. Day 25,** 5x **magnification.**
**Figure 18****. Comparative study of media including IMDM vs SLDM. Day 25, 10x magnification.**
**Figure 19****. Comparative study of media including IMDM vs SLDM. Day 25, 20x magnification.**
**Figure 20****. Comparative study of media including IMDM vs SLDM. Day 25, 5x magnification.**
**Figure 21****. Comparative study of media including IMDM vs SLDM. Day 25, 10x magnification.** Using traditional IMDM method, a greater number of non-neuronal "flat" cell types can be seen in the background using this method, when compared to SLDM culturing.
**Figure 22****. Comparative study of media including IMDM vs SLDM. Day 25, 20x magnification.**

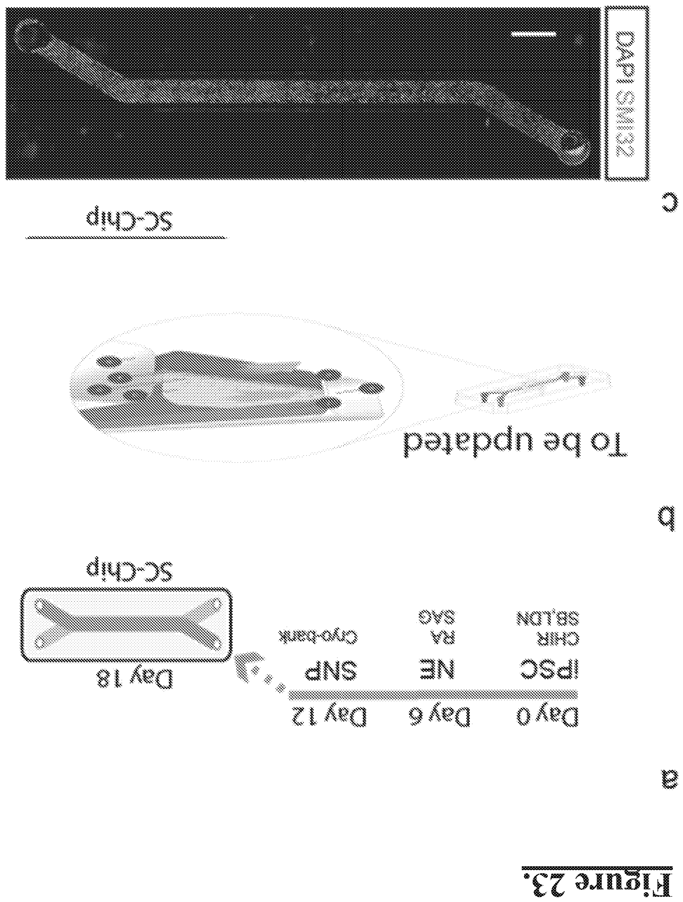

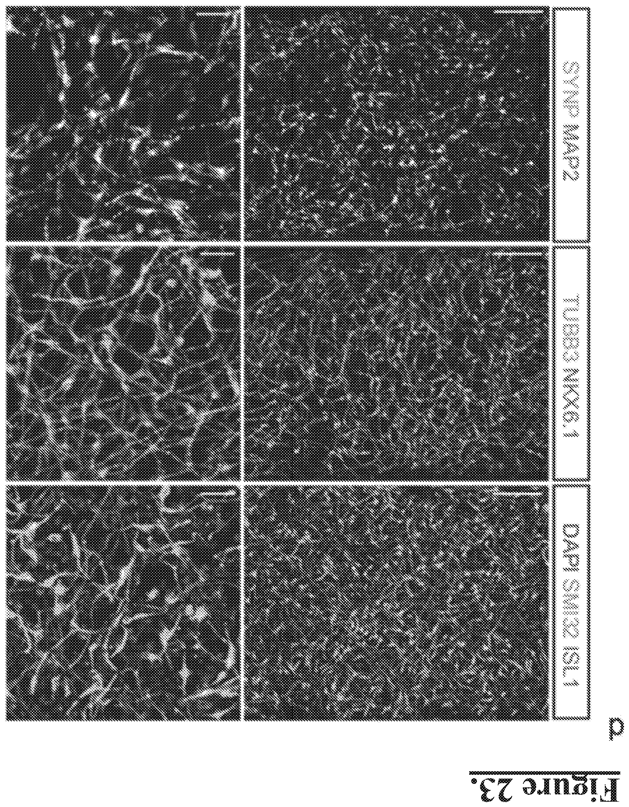

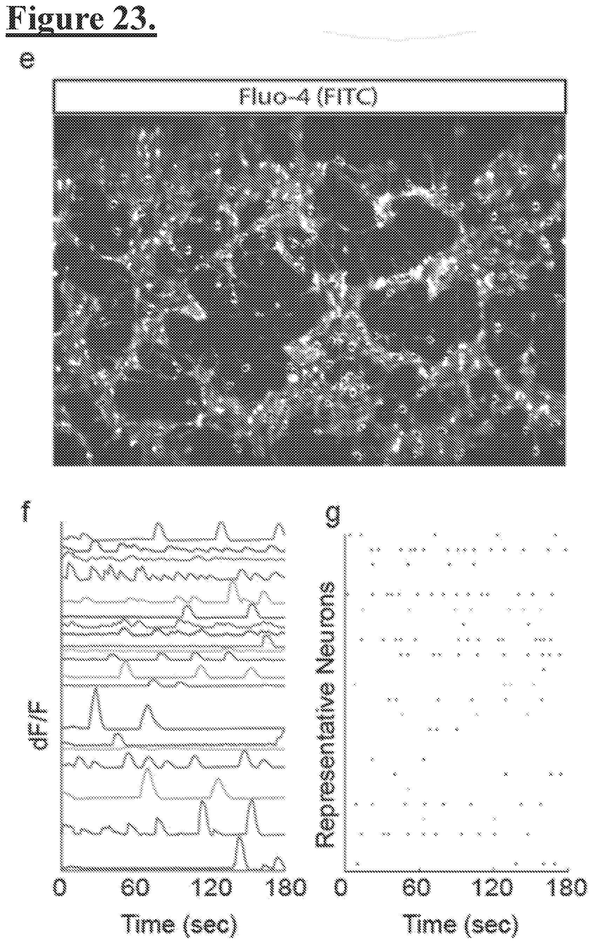

**Figure 23****: spNPCs survive and mature in the chip microenvironment.** (a) Schematic of Spinal neural progenitor (spNPC) differentiation from induced pluripotent stem cell (iPSC) cultures. Cells were fated to neural ectoderm (NE) using WNT agonist CHIR99021 and SMAD inhibitors LDN193189 and SB431542 for 6 days then patterned to ventral spinal neurons using Retinoic Acid (RA) and Sonic hedgehog agonist (SAG) in 6-well plates. At day 12, spNPCs were frozen, banked and thawed for experiments (Cryo-bank). spNPCs were seeded into the top channel of the SC-chip (dotted line) and incubated for 6 days. (b) Schematic of dual-channel organ chip seeded with spNPCs in top channel. (c) Phosphorylated neurofilament heavy chain (SMI32) is enriched in spinal motor neurons (spMNs) and expressed in cells populating entire top channel. Scale bar = 200 microns. (d) Immunostaining of main channel of the chip of markers of spMNs SMI32, nuclear marker isletl (ISL1), Beta 3 tubulin (TUBB3), NKX6.1, neurofilament marker microtubule-associated protein 2 (MAP2), and synaptic marker synaptophysin (SYNP), scale = 200 microns (left), 40 microns (right). (e) Representative image of SC-chip neurons treated with Fluo-4 calcium activated dye and acquired live in FITC channel. (f) Florescence of individual active neurons normalized to baseline florescence and plotted over time (dF/F) (g) Raster plot showing detected events of corresponding traces.

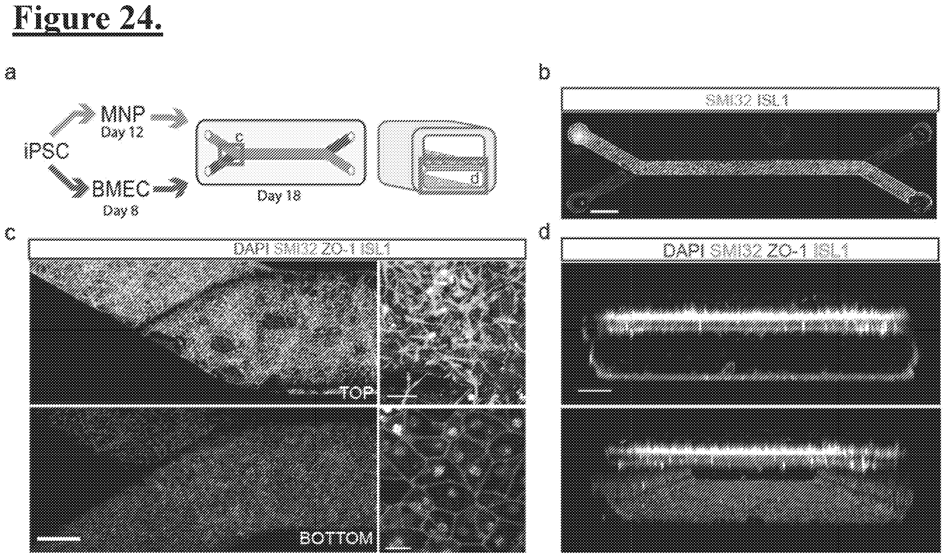

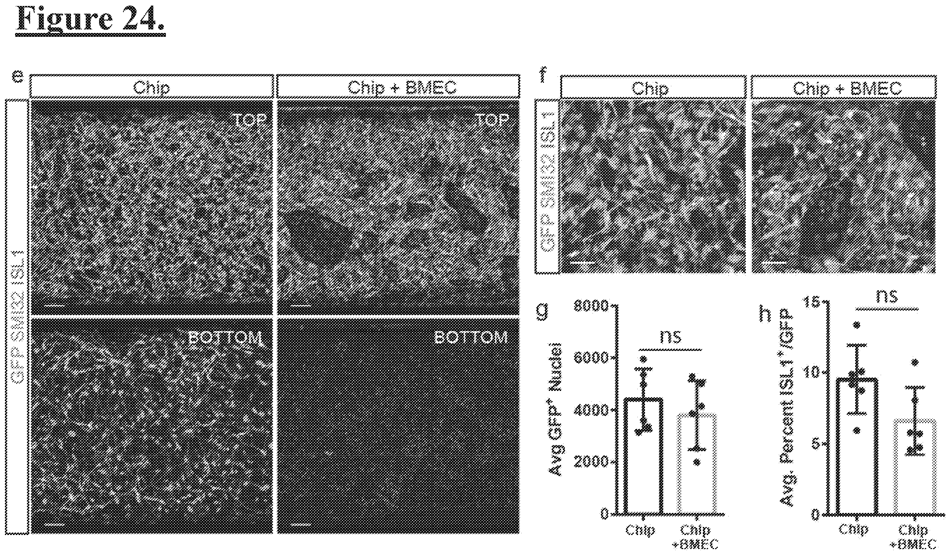

**Figure 24****: Co-culture of iPSC derived MNs and BMECs in Spinal Cord-Chip.** (a) Schematic of dual-differentiation and seeding paradigm in EC/Spinal Cord-Chip. Both spNPCs and BMECs are generated from human iPSCs and seeded into top and bottom channels respectively. Transverse section of the EC/Spinal Cord-Chip (right) shows two compartments separated by porous membrane. (b) Immunostaining of whole Spinal Cord-Chip at 6-days incubation expressing SMI32 in top channel and ISL1 expressed by BMECs on bottom channel. (c) Maximum projection images cropped along Z-axis to show top and bottom compartments of seeding end of EC/Spinal Cord-Chip immunostained with MN markers SMI32 and islet 1 (ISL1), and tight junction marker zona occludens 1 (ZO-1), Scale bar = 400 microns (left) 40 microns (right). (d) Confocal optical reconstruction at along Z axis (top) of Spinal Cord-Chip exhibits distinct separation of cultures at 6 days separated by porous PDMS membrane. Perspective view (bottom) exhibits confluent layer of BMECs surrounding entire bottom channel, Scale bar = 100 microns. (e) Representative images of cells attached to porous membrane in the main channel (top and bottom planes). Spinal Cord-Chip was seeded either with spNPCs alone in the top channel (Spinal Cord-Chip) or with the addition of non-GFP BMECs into the bottom compartment (EC/Spinal Cord-Chip). (f) High magnification of top compartment show GFP negative, ISL1 positive clusters indicating BMEC infiltration. (g-h) Quantification of GFP and ISL1 populations in top compartment. Error bars represent standard deviation. Plots were determined from 6 individual chips from three culture rounds (dots). No significant difference cultures determined by t-test (ns).

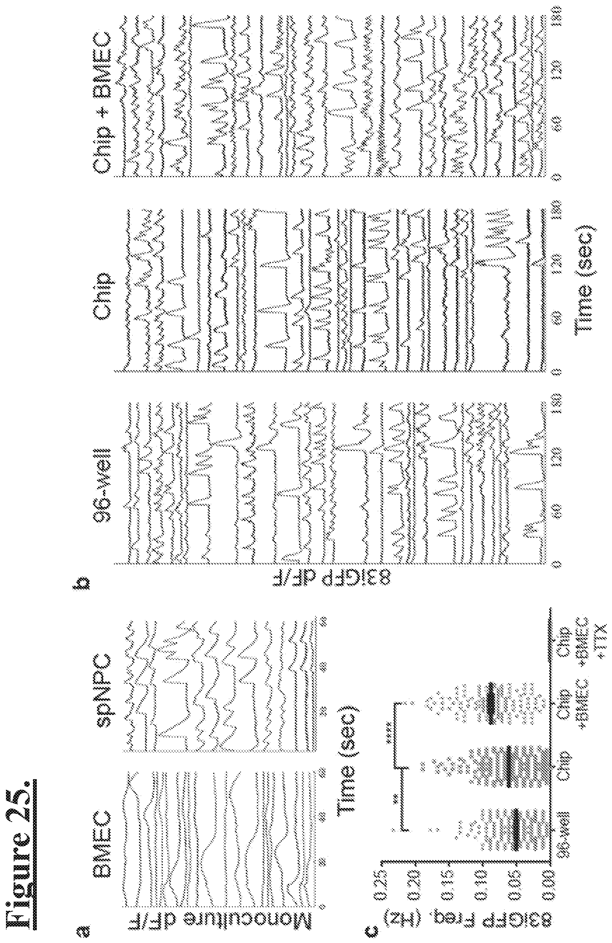

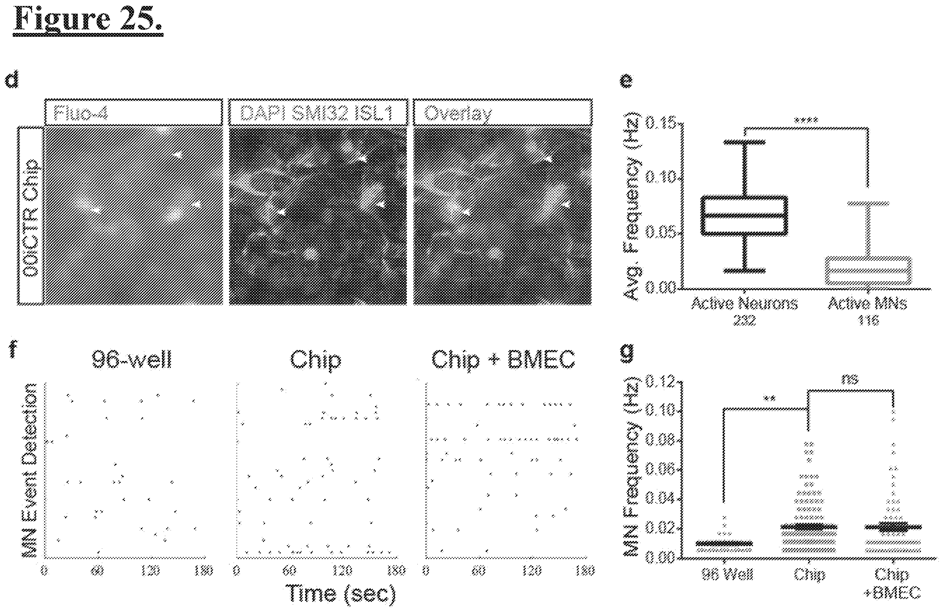

**Figure 25****: Spinal Cord-Chip environment increases spontaneous neural activity.** (a) Individual Representative calcium transient activity of spNPC (top) and BMEC (bottom) cultures acquired at the seeding compartments where cultures do not interact. Change in florescence intensity over background (dF/F) plotted with respect to time totaling 60 seconds. (b) Calcium transient activity plots of 30 representative neurons derived from 83iGFP spNPC cultures in each culture condition. (c) Transient frequency of 194-297 neurons spanning three separate replicates per condition. Neuron activity was completely ablated with the administration of tetrodotoxin (TTX). (d) Immunocytochemistry staining of isletl (ISL1) and smi32 (right) of site previously acquired for live calcium transients using fluro-4 dye (left). ISL1⁺ neurons (arrows) superimposed to determine MN firing specificity. (e) Mean transient frequency of representative active neurons derived from 00iCTR spNPC cultures compared to ISL1 overlaid MNs. (g-h) 30 Representative transient events from ISL1 SMI32 double-positive neurons quantified and displayed as raster plot and plotted as frequency in hertz (Hz). Means represented by black bars and error bars represent standard error of the mean. Significance calculated by one way ANOVA, ^{∗∗} denotes P < 0.01, ^{∗∗∗} = P < 0.005, and ^{∗∗∗∗} = P < 0.001.

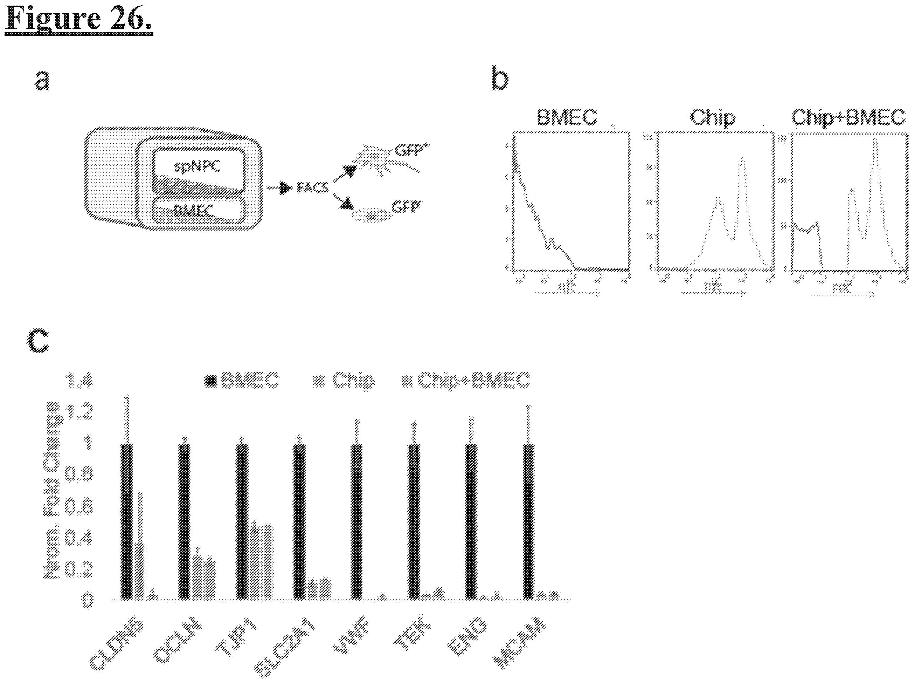

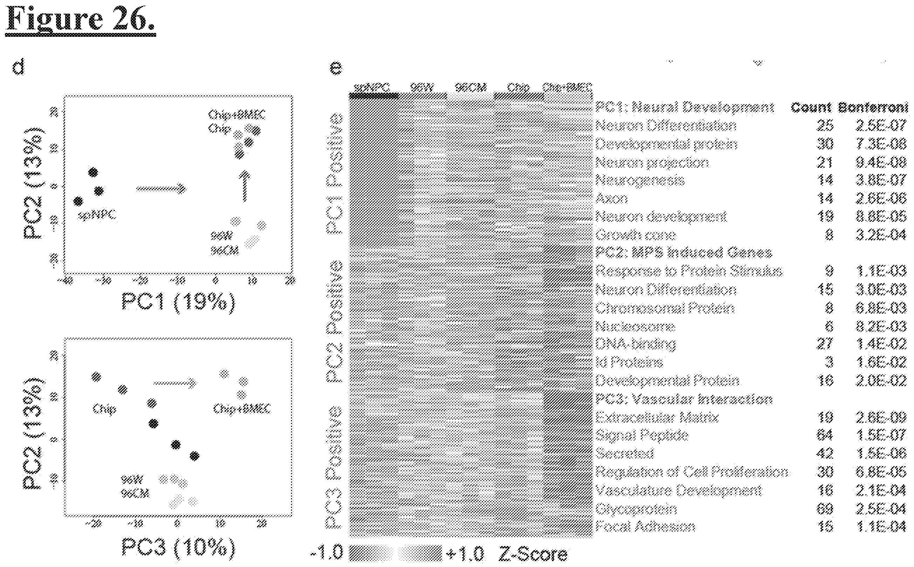

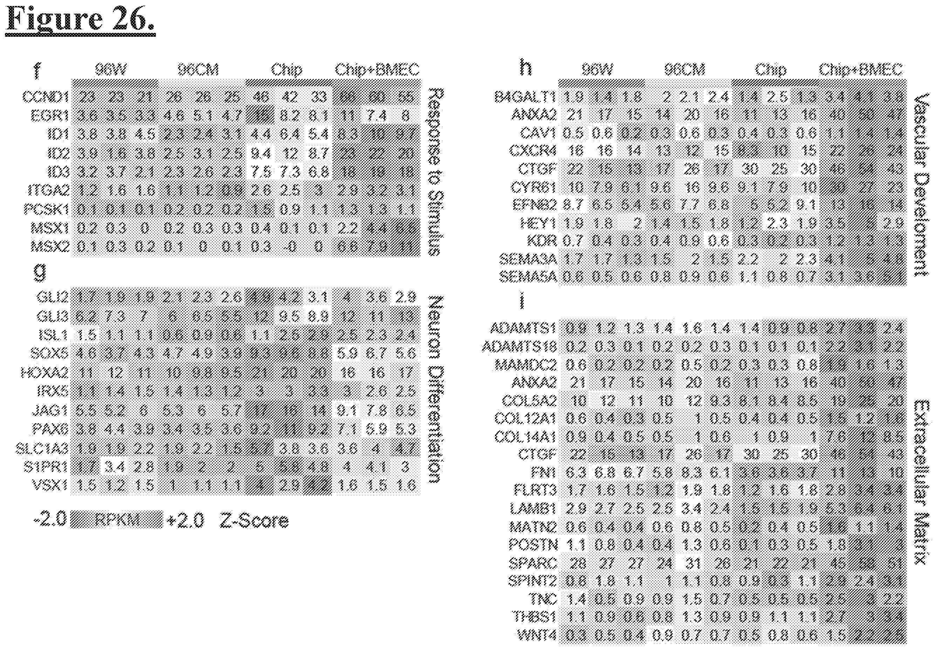

**Figure 26****: Spinal Cord-Chip induces neural differentiation and vascular interaction gene expression.** (a) GFP-MN isolation schematic: nuclear GFP expressing spNPCs seeded on top channel with isogenic non-GFP expressing BMECs on bottom. Fluorescence-assisted cell sorting (FACS) conducted on FITC to purify spMNs after BMEC co-culture. (b) Single population histogram of chips seeded only with non-GFP BMECs (black) or GFP-MNs (green) that defined negative and positive fractions respectively. Number of events displayed on y axis and FITC intensity on x-axis (c) Mean RPKM data of purified Spinal Cord-Chip and EC/Spinal Cord-Chip cultures normalized to RPKM data from iPSC derived BMECs cultured alone. Canonical markers claudin 5 (CLDN5), occluding (OCLN), Tight junction protein 1 (TJP1), Glut-1 (SLC2A1), von willebrand factor (VWF), Tie2 receptor (TEK), endoglin (ENG), and melanoma cell adhesion molecule (MCAM). (d) Principle component analysis plots of first two principle components (PC) (top) and PCs 2, and 3 (bottom). Arrows indicate weighting along the axis of each respective PC. (e) Top 200 ranked genes from each PC displayed as Z-score calculated across all conditions for each row and indicated by colorimetric scale. Each PC was entered into DAVID ontology pathway analysis and top 7 categories listed for each PC. The number of genes (count) in each category are displayed with corrected significance values from DAVID analysis (Bonferonni). (f,g) Differentially expressed genes contributing to the "Response to Protein Stimulus" and "Neural Differentiation" DAVID terms enriched in PC2. RPKM data averaged across sample replicates and normalized to 96W control. (h,i) Differentially expressed genes contributing to "Vascular Development" and "Extracellular Matrix" DAVID terms enriched in PC3.

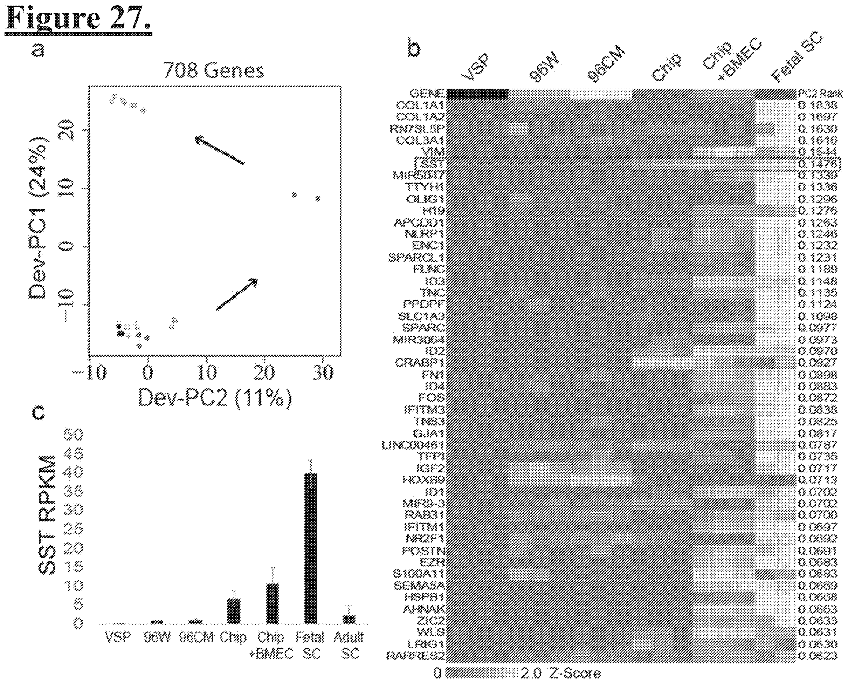

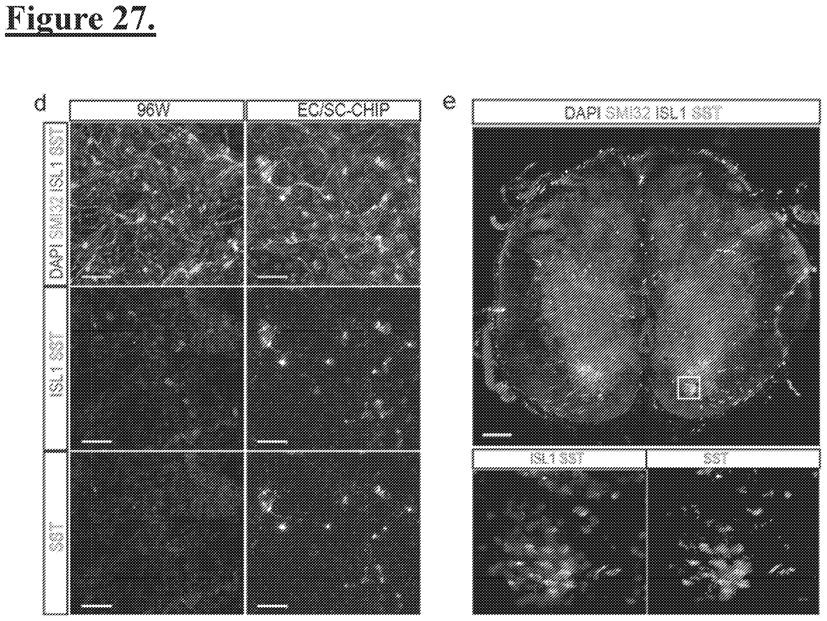

**Figure 27****: BMECs induce vascular interaction gene expression in Chip.** (a) PCA comparing expression data of differentially expressed genes from PC2 and PC3 and including in vivo adult laser captured MNs (green), in vivo fetal spinal cord (purple), and in vitro experimental data. (b) Heatmap of top ranking genes from Dev-PC2 that describe variance in fetal gene expression. Z-score calculated from Log2 RPKM data per gene row and displayed by colorimetric scale. (c) Mean Somatostatin (SST) expression plotted for each condition, error bars represent standard deviation. (d) Representative images of 96 well (96W) and Spinal Cord-Chips co-cultured with BMECs (EC/Spinal Cord-Chip) immunostained with SST and MN markers SMI32 and ISL1, scale bars = 40 microns. (e) Whole mount image of day 67 human fetal spinal cord (top) immunostained with SST, SMI32 and ISL1. Higher magnification of ventral horn ISL1 positive MN pool (box) co-expressing SST (bottom).

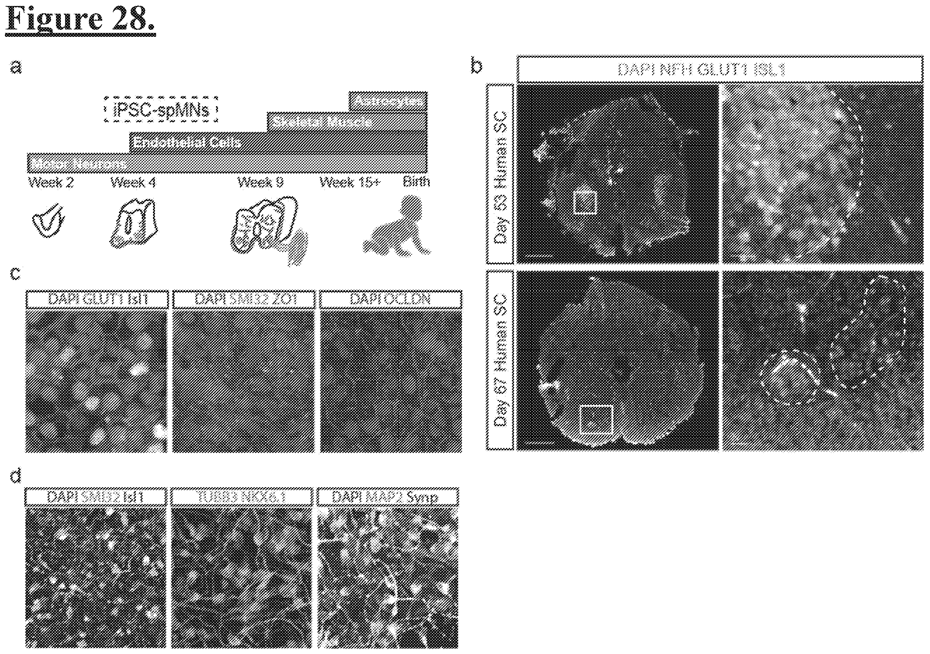

**Figure 28:** (a) Schematic of relative emergence of cell types that directly interact with developing human MNs in vivo. (b) Days 53 and 67 human fetal spinal cords immunostained with neuronal marker neuofilament heavy chain (NFH), BMEC marker glucose transporter 1 (GLUT1), and transcription factor ISL1. High magnification of ventral horns (box) show ISL1 positive MN pools (yellow dotted line). (c) Images of iPSC-derived BMECs cultured in 96-well plate for 6 days and immunostained with GLUT1, ISL1, and tight junction markers zona occludens 1 (ZO-1) and Occludin (OCLDN). (d) Images of spNPCs cultured in 96W plate and immunostained with markers of spMNs SMI32, nuclear marker isletl (ISL1), Beta 3 tubulin (TUBB3), NKX6.1, neurofilament marker microtubule-associated protein 2 (MAP2), and synaptic marker synaptophysin (SYNP).
**Figure 29:** (a) Calcium transient activity plots of 30 representative neurons derived from 00iCTR spNPC cultures in each culture condition. (b) Quantification of at least 3 chips per condition from one experimental replicate.
**Figure 30:** (a) Density plots of 10,001 genes for each sample for transcriptomic analysis. Quantile normalized data (right). (b) Pierson correlation coefficient analysis of normalized RPKM datasets displayed by colorimetric scale. (c) Notch pathway genes differentially expressed in the Spinal Cord-Chip conditions.
**Figure 31:** (a) Density plots of 10,001 genes of developmental comparison containing adult laser captured MNs (green), fetal Spinal cord (purple) and sorted iPSC-derived neurons (black). Quantile normalized data (bottom). (b) PCA analysis of variance across all samples and all 10,001 genes.

### DETAILED DESCRIPTION

Unless defined otherwise, technical and scientific terms used herein have the same meaning as commonly understood by one of ordinary skill in the art to which this invention belongs. Allen et al., Remington: The Science and Practice of Pharmacy 22nd ed., Pharmaceutical Press (September 15, 2012); Hornyak et al., Introduction to Nanoscience and Nanotechnology, CRC Press (2008); Singleton and Sainsbury, Dictionary of Microbiology and Molecular Biology 3rd ed., revised ed., J. Wiley & Sons (New York, NY 2006); Smith, March's Advanced Organic Chemistry Reactions, Mechanisms and Structure 7th ed., J. Wiley & Sons (New York, NY 2013); Singleton, Dictionary of DNA and Genome Technology 3rd ed., Wiley-Blackwell (November 28, 2012); and Green and Sambrook, Molecular Cloning: A Laboratory Manual 4th ed., Cold Spring Harbor Laboratory Press (Cold Spring Harbor, NY 2012), provide one skilled in the art with a general guide to many of the terms used in the present application. For references on how to prepare antibodies, see Greenfield, Antibodies A Laboratory Manual 2nd ed., Cold Spring Harbor Press (Cold Spring Harbor NY, 2013); Köhler and Milstein, Derivation of specific antibody-producing tissue culture and tumor lines by cell fusion, Eur. J. Immunol. 1976 Jul, 6(7):511-9; Queen and Selick, Humanized immunoglobulins, U. S. Patent No. 5,585,089 (1996 Dec); and Riechmann et al., Reshaping human antibodies for therapy, Nature 1988 Mar 24, 332(6162):323-7.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

Some abbreviations are used herein. For example, "MN" refers to motor neurons. The letter "i" indicates "induced." Thus, "iMN" indicates induced motor neurons, i.e. motor neurons that were induced or generated from other cells, e.g. stem cells. "diMN" indicates direct induced motor neurons. "iMNP" indicates induced motor neuron progenitor cells, which are not fully differentiated into mature neurons.

There are many ways to evaluate the integrity and physiology of an in vitro system that mimics the blood brain barrier. Two of the most common methods are Transepithelial Electric Resistance (TEER) and Lucifer Yellow (LY) rejection. Importantly, manipulations must be
performed using aseptic techniques in order for the cells to remain in culture without contamination. TEER measures the resistance to pass current across one or more cell layers on a membrane. The measurement may be affected by the pore size and density of the membrane, but it aims to ascertain cell and/or tissue properties. The TEER value is considered a good measure of the integrity of the cell monolayer.

Lucifer Yellow (LY) travels across cell monolayers only through passive paracellular diffusion (through spaces between cells) and has low permeability. Therefore it is considerably impeded in passing across cell monolayers with tight junctions. Permeability (Papp) for LY of ≤ 5 to 12 nm/s has been reported to be indicative of well-established monolayers.

A wide variety of human tissues can be generated from human induced pluripotent stem cells (iPSCs). However, cells generated *in vitro* in most culture systems remain fetal-like in nature, thereby limiting their utility for research and disease modeling. Comparative analysis of developing spinal cord tissue and iPSC-derived motor neuron cultures have revealed that even with prolonged time in culture, spinal motor neurons generated from iPSCs do not possess functional and molecular maturation signatures beyond 4-6 gestational weeks. Novel culture systems that aim to enhance the function and maturation of iPSC-derived motor neurons (MNs) *in vitro* are of considerable interest, especially in order to study MN dysfunction in adult-onset disorders such as amyotrophic lateral sclerosis (ALS).

Unlike conventional cell culture plates, Organ-on-Chip systems offer 3-diminsional microengineered platforms that aim to better recapitulate the cellular microenvironment including cell-cell interactions at the microscale. Due to reduced media volume and non-convection geometry, microengineered cultures may provide greater concentration of soluble signals compared to 96-well plates. While groups have described these microvolume effects on various cell types, the neurogenic effects of microvolume culture on iPSC-derived neurons remain unknown, prompting us to explore them here.

Developing spMNs rely on other cell types to provide signaling cues critical to maturation. Neuromuscular junction formation and astrocyte emergence begin at 9 and 15 weeks' post-fertilization, respectively, and astrocytes continue to proliferate into post-natal development. While the importance of the interaction of these cell types in improving MN function has been demonstrated *in vitro,* this is not sufficient to fully mature MNs. Of note, preceding astrocyte emergence, brain microvascular endothelial cells (BMECs) invade the neural tube from the perineural vascular plexus beginning at 4 weeks post-fertilization and form a primitive blood brain barrier that directly interacts with developing neural tube progenitors and neurons. During development, vascular angiogenesis and axon neurite outgrowth share common morphogenic mechanisms known as angioneurins. In the adult context, BMECs have also been shown to influence neurogenesis. The concordant developmental timing of the two cell types, as well as shared developmental signaling pathways prompted the Inventors' hypothesis that iPSC-derived BMECS could interact with and mature iPSC-derived MN cultures *in vitro.* In order to explore this hypothesis, the Inventors used an Organ-Chip system that provide unique co-culture paradigms to allow distinct cell types to interact in a controlled manner.

In the current study, the Inventors use ventralized spinal neural progenitors, generated from human iPSCs in a Spinal Cord-Chip system. Compared to 96-well plate, cultures in the Chip had increased neural activity and expressed enhanced neural differentiation genes. When iPSC-derived BMECs were included, the Inventors observed further significant increases in neuronal activity, induction of vascular-neuron interaction genes, and developmental gene expression profile that indicated a more *in vivo*-like signature. This platform provides insight into endogenous signaling of spinal neural cultures and BMEC interaction during early human development. It also provides a novel vascularized *in vitro* model of motor neuron differentiation from iPSCs to better understand motor neuron-related diseases.

Described herein as an example, and not forming part of the invention, is a method of generating spinal neural progenitor cells (spNPCs) including providing induced pluripotent stem cells (iPSCs), differentiating iPSCs into neural ectodermal cells by culturing in neural induction media, and dissociating neural ectodermal cells, replating on a cell culture substrate and further culturing the replated neural ectodermal cells in differentiation media to generate spNPCs. In other examples, the iPSCs are cultured in neural induction media for a period of about 5-7 days, including about 6 days. In other examples, the replated neural ectodermal cells are cultured in differentiation media for a period of about 5-7 days, including about 6 days. In other examples, the neural induction media comprises one or more of: LDN193189, SB431542, and CHIR99021. In other examples, the cell culture substrate is matrigel. In other examples, the differentiation media comprises one or more of: ascorbic acid, retinoic acid, SAG. In other examples, the method further includes freezing the spNPCs. In various examples, the concentration of LDN193189 is 0.02 to 2 uM, SB431542 is 0.1 to 100 um, and CHIR99021 is 0.3 uM to 30 uM. In various examples, the concentration of retinoic acid is 0.01 um to 2 uM, , cAMP is 0.01 uM to 5 uM, SAG is 0.01 uM to 5 uM, GDNF is 1 to 50 ng/mL, BDNF is 1 to 50 ng/mL.

For example, iPSCs mechanically passaged at low density, can be differentiated for 6 days to drive neural ectodermal cells in neural induction media consisting of IMDM/F12, B27, N2, 1% NEAA, 0.2 uM LDN193189, 10uM SB431542, 3uM CHIR99021. Alternatively, SLDM, without B27, N2, 1% NEAA, 0.2 uM LDN193189, 10uM SB431542, 3uM CHIR99021 may be used. Cells are then passaged with enzymatic dissociation, such as using Accutase and reseeded onto a cell culture substrate such as matrigel. In some instances, one may pattern the neural ectodermal cells to form spNPCs in Stage 2 media consisting of IMDM/F12, B27, N2, 1% NEAA, 200ng/ml ascorbic acid, 0.1 uM retinoic acid, 1 uM SAG for an additional 6 days. Alternatively, SLDM, without B27, N2, 1% NEAA, 200ng/ml ascorbic acid, 0.1 uM retinoic acid, 1 uM SAG may be used. spNPC cells can then be dissociated and cryogenically frozen for later use. Upon thaw, MNs were cultured in Stage 3 media consisting of IMDM/F12, B27, N2, 1% NEAA, 200ng/ml ascorbic acid, 0.5 uM retinoic acid, 0.1 uM cAMP, 0.1 uM SAG, lOng/ml GDNF, 10 ng/ml BDNF, 1% PSA and fed every two days. Alternatively, SLDM without B27, N2, 1% NEAA, 200ng/ml ascorbic acid, 0.5 uM retinoic acid, 0.1 uM cAMP, 0.1 uM SAG, lOng/ml GDNF, 10 ng/ml BDNF, 1% PSA can be used. At day 12 of culture cells are labeled ventral spinal neuron progenitors (SNP) when cultured using the method described in this and the above paragraph. At day 12, SNPs may be frozen, banked and then thawed when used for seeding chips or used to directly seed chips.

Also described herein as an example, and not forming part of the invention is a cryopreserved solution of spNPCs made by is a method of generating spinal neural progenitor cells (spNPCs) including providing induced pluripotent stem cells (iPSCs), differentiating iPSCs into neural ectodermal cells by culturing in neural induction media, and dissociating neural ectodermal cells, replating on a cell culture substrate and further culturing the replated neural ectodermal cells in differentiation media to generate spNPCs.

Described herein as an example, and not forming part of the invention, is a cell culture supplement, including one or more first additives selected from the group consisting of: Albumin, Bovine Serum, Sodium Bicarbonate, L-Ascorbic Acid, Putrescine, D(+)-Galactose, Holo-transferrin, Catalase, L-Carnitine, Glutathione (reduced), Sodium Selenite, Ethanolamine, and T3 (triiodo-L-thyronine), and one or more second additives selected from the group consisting of: Corticosterone, Linoleic Acid, Linolenic Acid, Lipoic Acid (thioctic acid), Progesterone, Retinol Acetate, a-Tocopherol (vitamin E), and a-Tocopherol acetate. Further described herein is a culture media including the cell culture supplement and a base media. In other examples, the base media comprises DMEM/F12. For example, a cell culture supplement including one or more first additives, and one or more second additives includes all of the additives listed in Table 1. In various examples, the additives are at a concentration of 0.01-10, 10-100, 100-500, 500 or more ug/mL. In other examples, the additives are at a concentration of 1-10, 10-50, 50-100, 100 or more mg/mL. Such supplement may be added in a base media, including DMEM/F12 and other additives as shown in Table 2.

Also described herein as an example, and not forming part of the invention, is a method of generating spinal neural progenitor cells (spNPCs) including providing induced pluripotent stem cells (iPSCs), differentiating iPSCs into neural ectodermal cells by culturing in neural induction media for about 6 days, and dissociating neural ectodermal cells, replating on a cell culture substrate and further culturing the replated neural ectodermal cells in differentiation media for about 6 days to generate spNPCs. In other examples, the neural induction media, differentiation media, or both, comprise a supplement including: one or more first additives selected from the group consisting of: Albumin, Bovine Serum, Sodium Bicarbonate, L-Ascorbic Acid, Putrescine, D(+)-Galactose, Holo-transferrin, Catalase, L-Carnitine, Glutathione (reduced), Sodium Selenite, Ethanolamine, and T3 (triiodo-L-thyronine), and one or more second additives selected from the group consisting of: Corticosterone, Linoleic Acid, Linolenic Acid, Lipoic Acid (thioctic acid), Progesterone, Retinol Acetate, a-Tocopherol (vitamin E), and a-Tocopherol acetate. In other examples, the neural induction media comprises one or more of: LDN193189, SB431542, and CHIR99021. In other examples, the cell culture substrate is matrigel. In other examples, the differentiation media comprises one or more of: ascorbic acid, retinoic acid, SAG. Also described herein is a quantity of spNPCs , made by the aforementioned method. In other examples, the method further includes freezing the spNPCs. Further described herein is a a cryopreserved solution of spNPCs made by the aforementioned method.

### Example 1

### Pluripotent stem cell culture, directed differentiation into spMNs and BMECs:

Two human induced pluripotent stem cell lines (83iCTR and 00iCTR) used in this study were previously derived from non-diseased control patient fibroblasts. Spinal motor neuron derivation was achieved using iPSCs mechanically passaged at low density, then differentiated for 6 days to drive neural ectodermal cells in neural induction media consisting of IMDM/F12 (Gibco), B27, N2, 1% NEAA, 0.2 uM LDN193189, 10uM SB431542, 3uM CHIR99021 (Cayman Chemical). Cells were then passaged using Accutase (Sigma-Aldrich) and reseeded onto matrigel and patterned to form spNPCs in Stage 2 media consisting of IMDM/F12 (Gibco), B27, N2, 1% NEAA, 200ng/ml ascorbic acid, 0.1 uM retinoic acid, 1 uM SAG for an additional 6 days. Cells were then dissociated and cryogenically frozen for later use. Upon thaw, MNs were cultured in Stage 3 media consisting of IMDM/F12 (Gibco), B27, N2, 1% NEAA, 200ng/ml ascorbic acid, 0.5 uM retinoic acid, 0.1 uM cAMP, 0.1 uM SAG, lOng/ml GDNF, 10 ng/ml BDNF, 1% PSA and fed every two days. At day 12 of culture cells are labeled ventral spinal neuron progenitors (SNP) when cultured using the method described in this and the above paragraph. At day 12, SNPs may be frozen, banked and then thawed when used for seeding chips or used to directly seed chips.

For BMEC Differentiation, cells are thawed (or dissociated fresh) and seeded into the chip at day 8-9 (in the case of BMECs differentiation) and at various points in neural differentiation.

### Example 2

### Improved media formulation

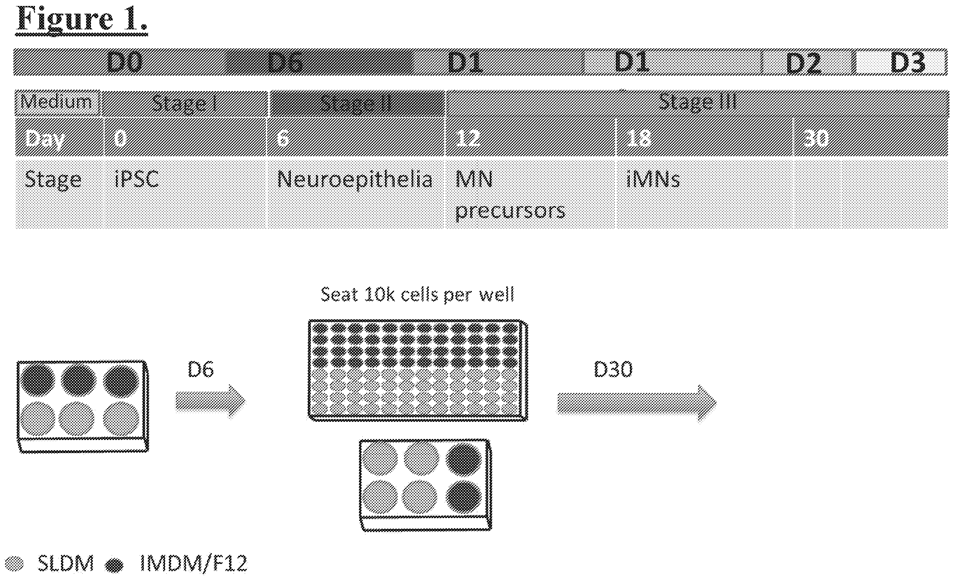

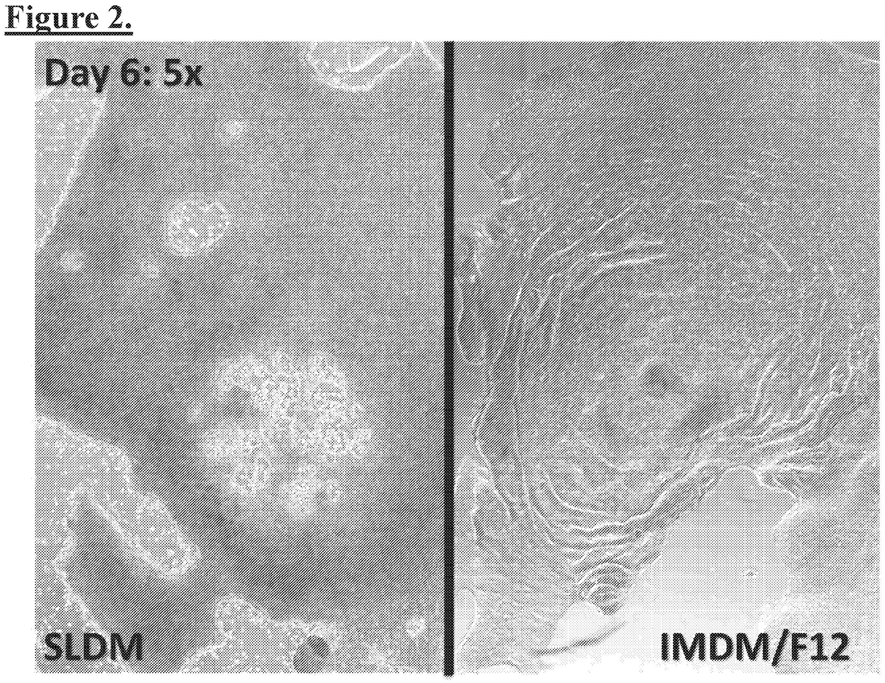

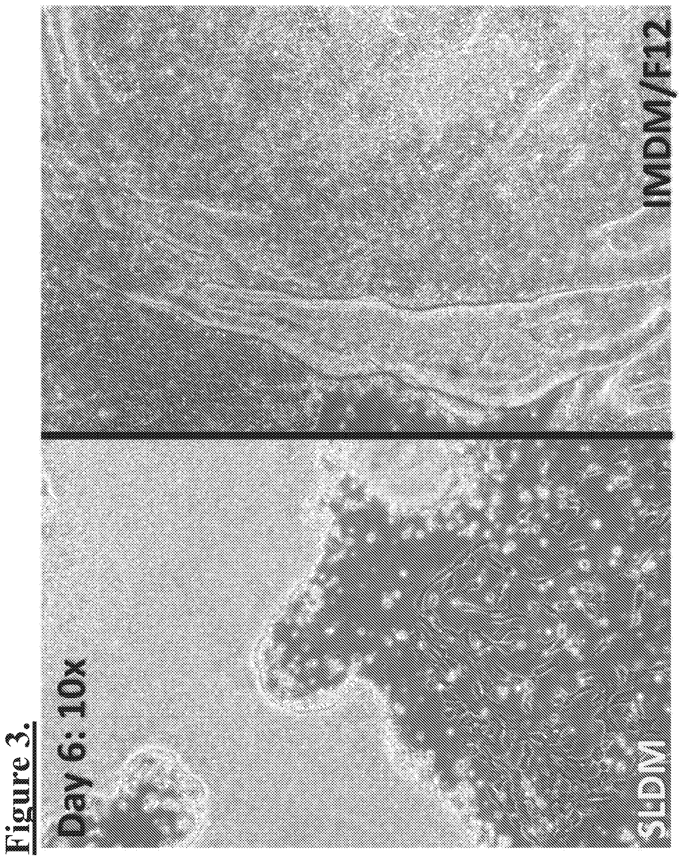

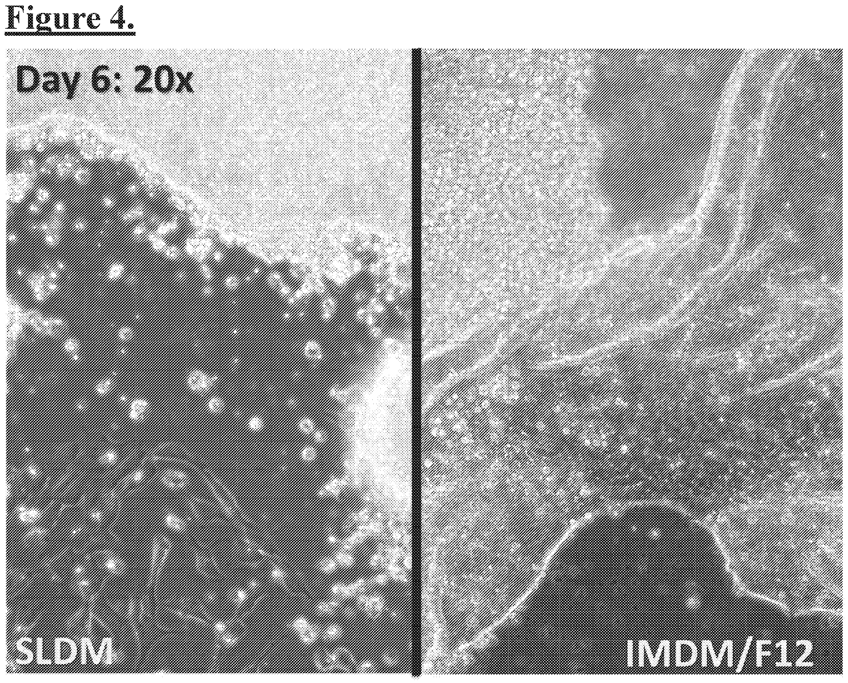

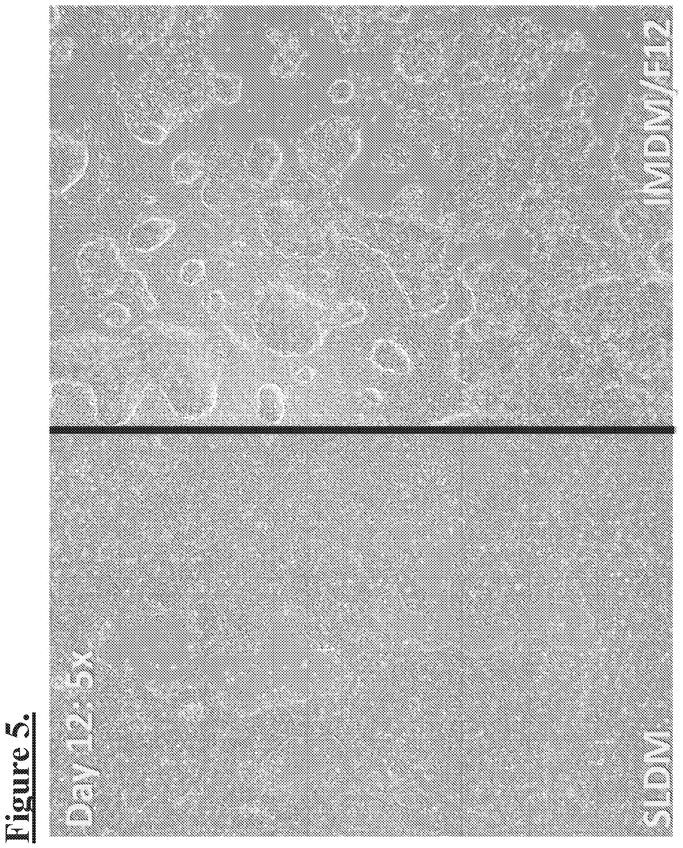

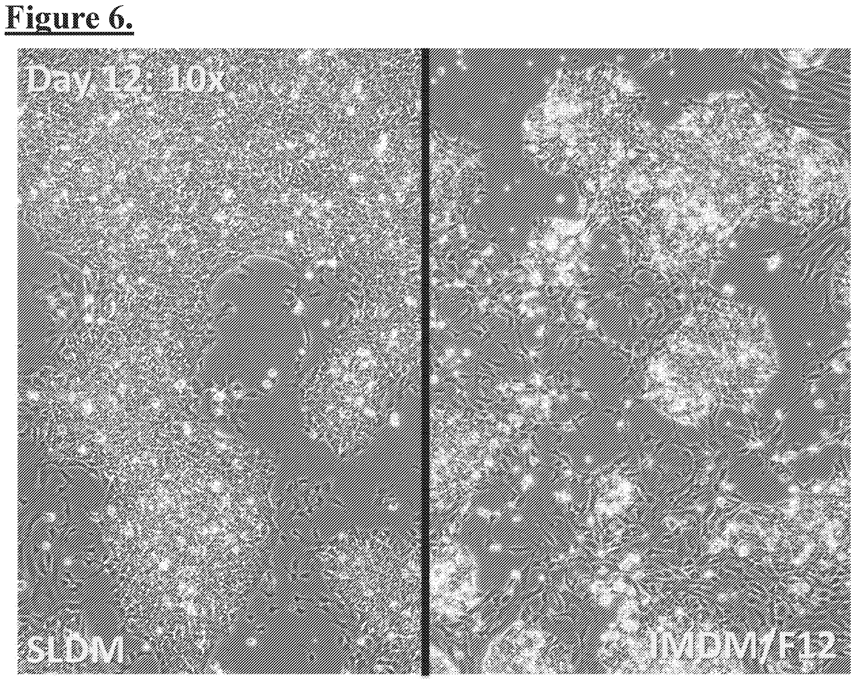

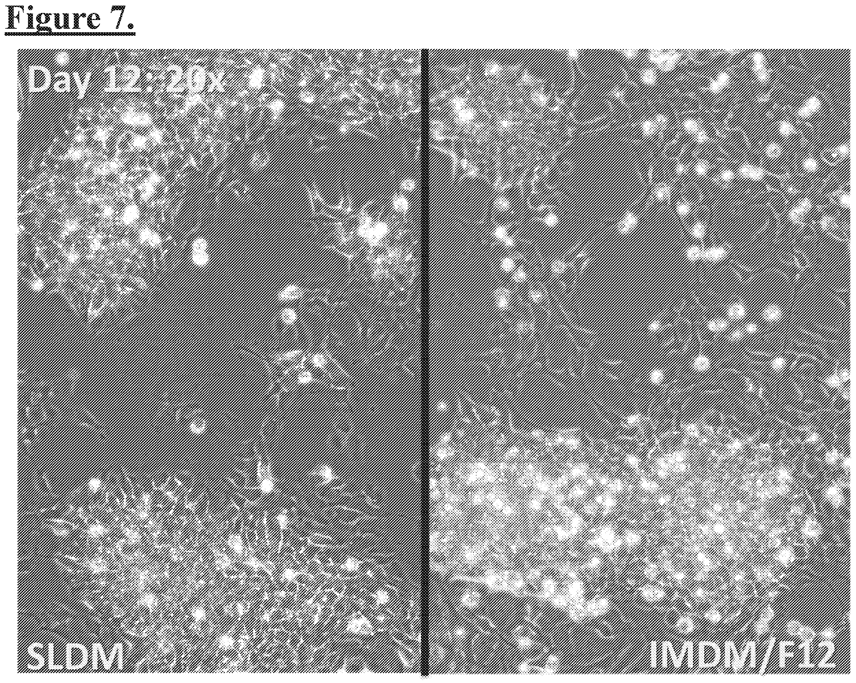

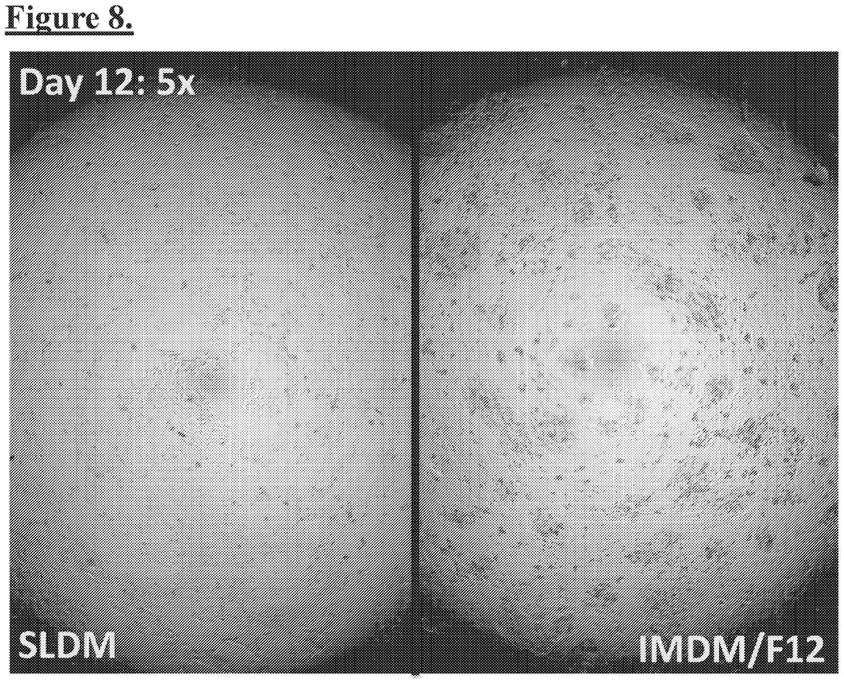

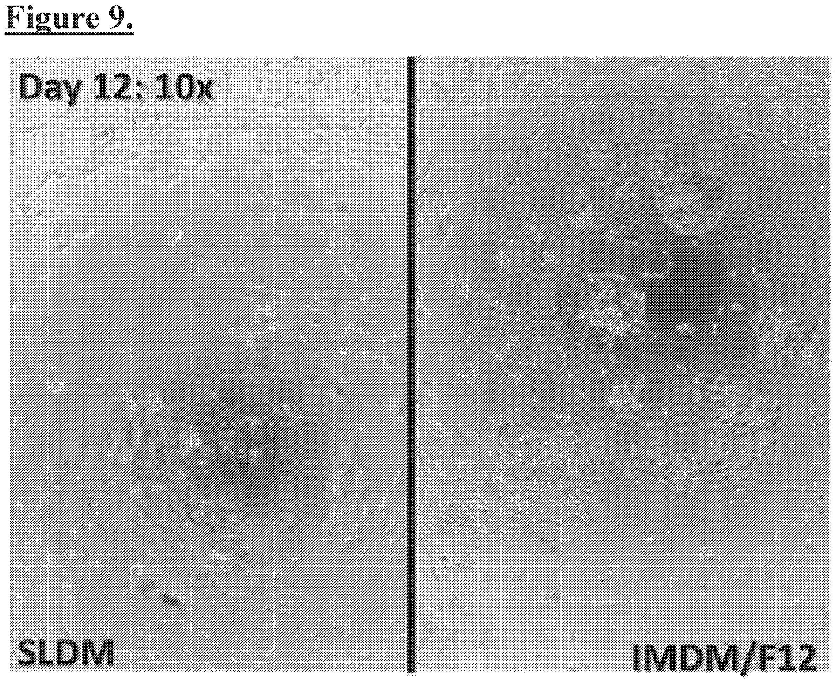

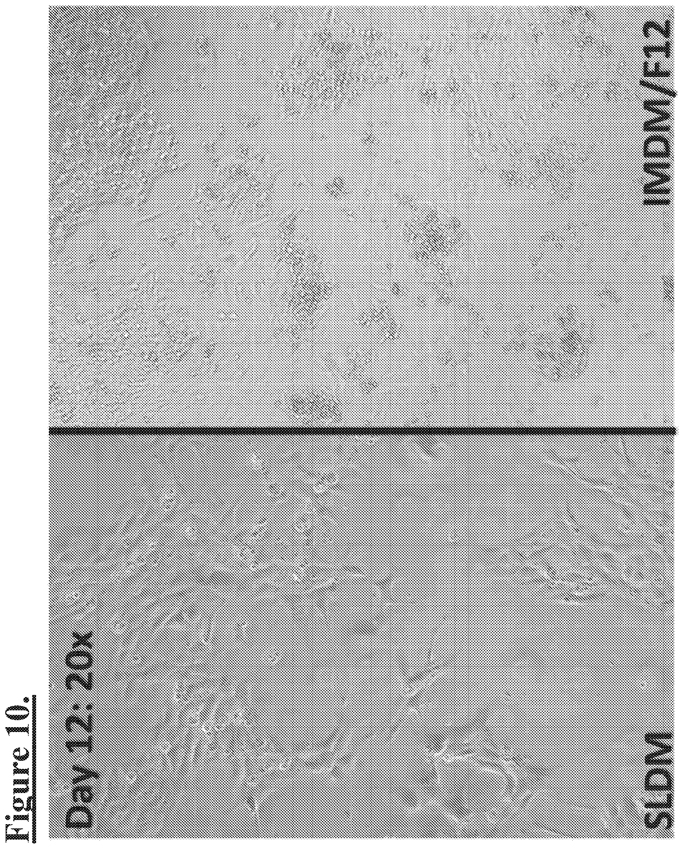

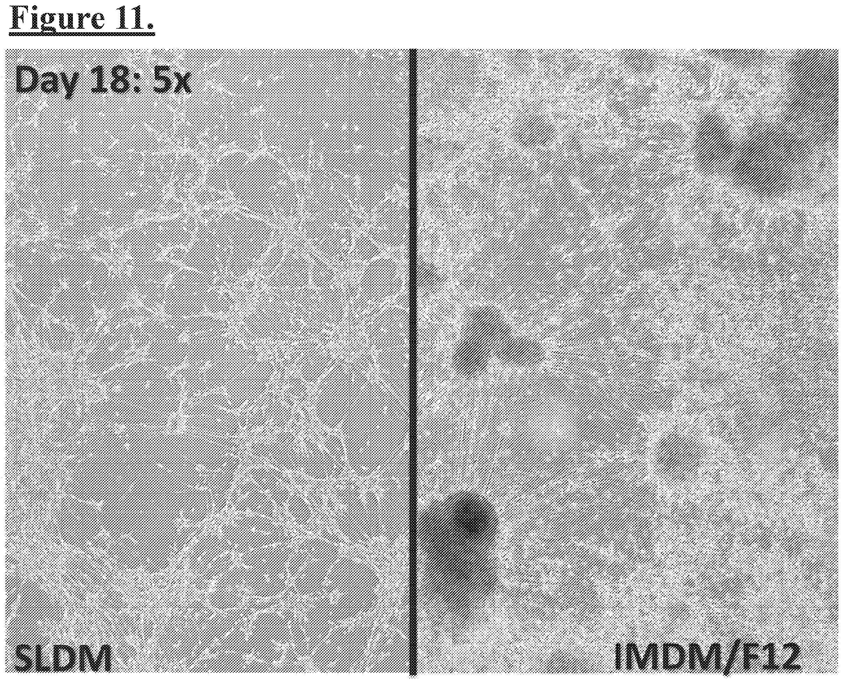

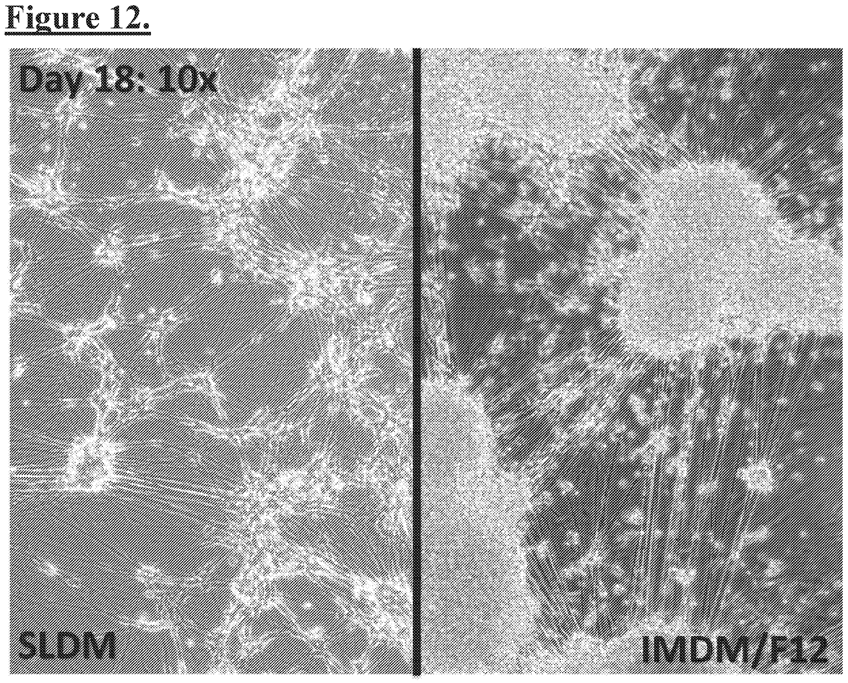

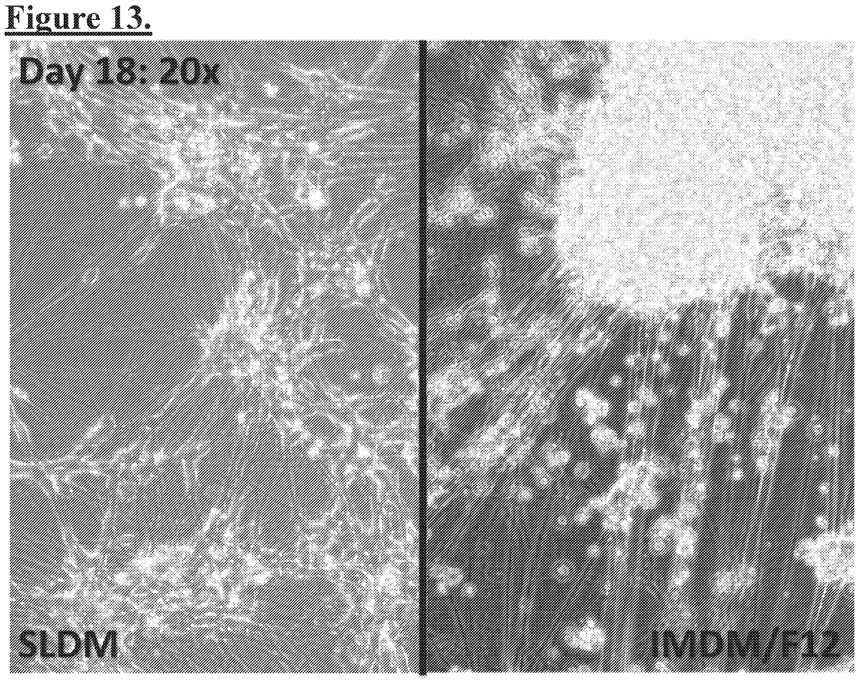

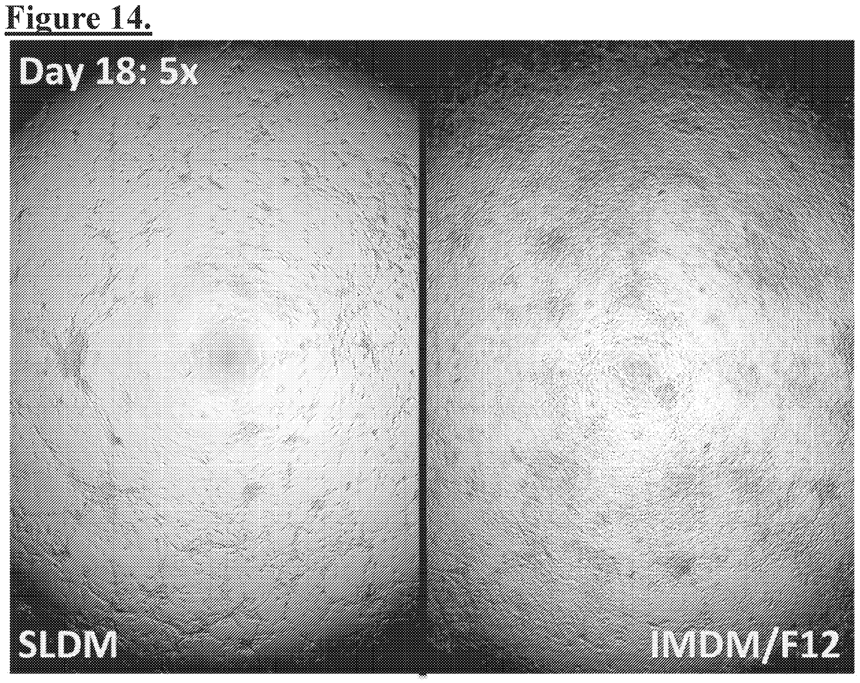

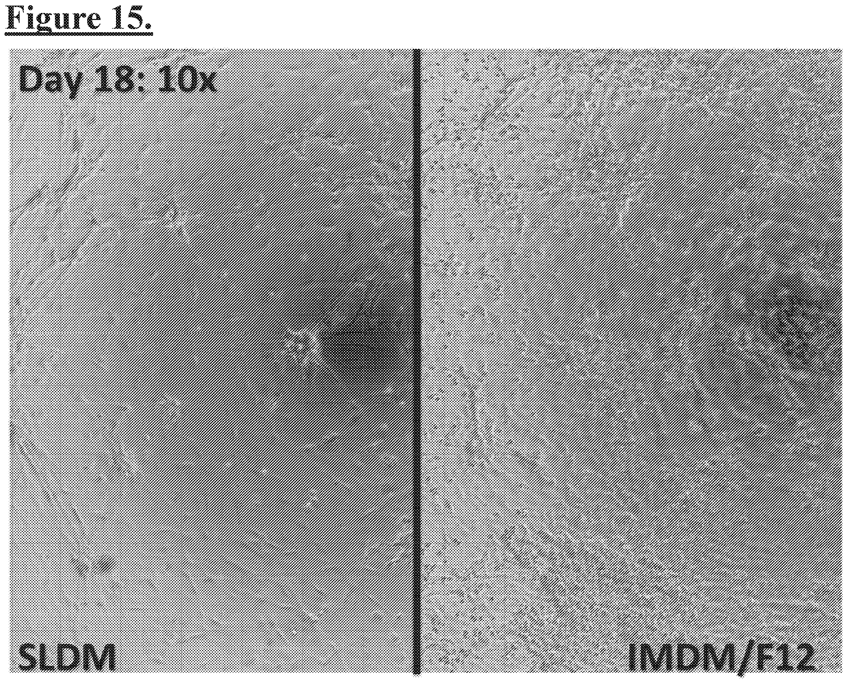

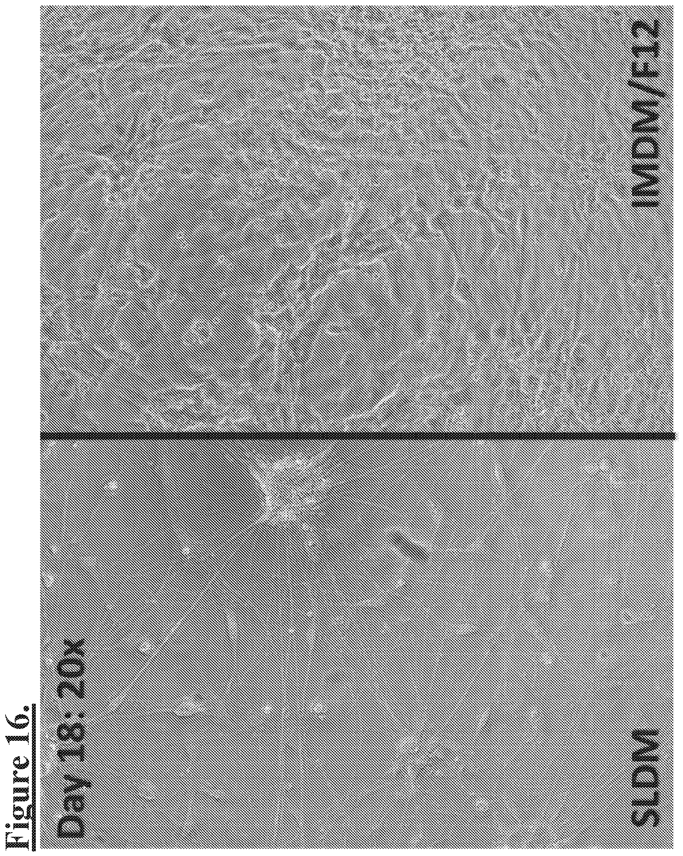

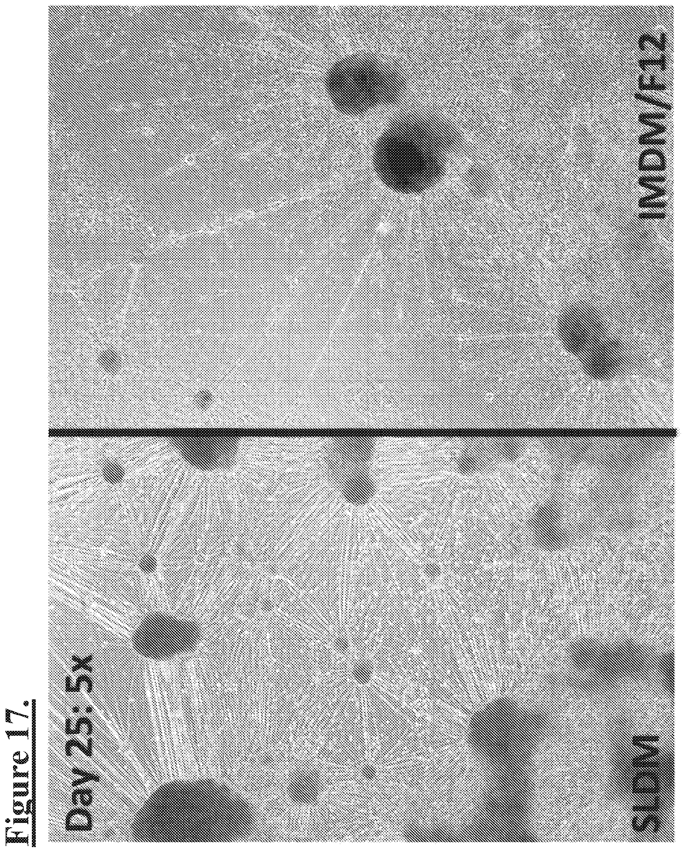

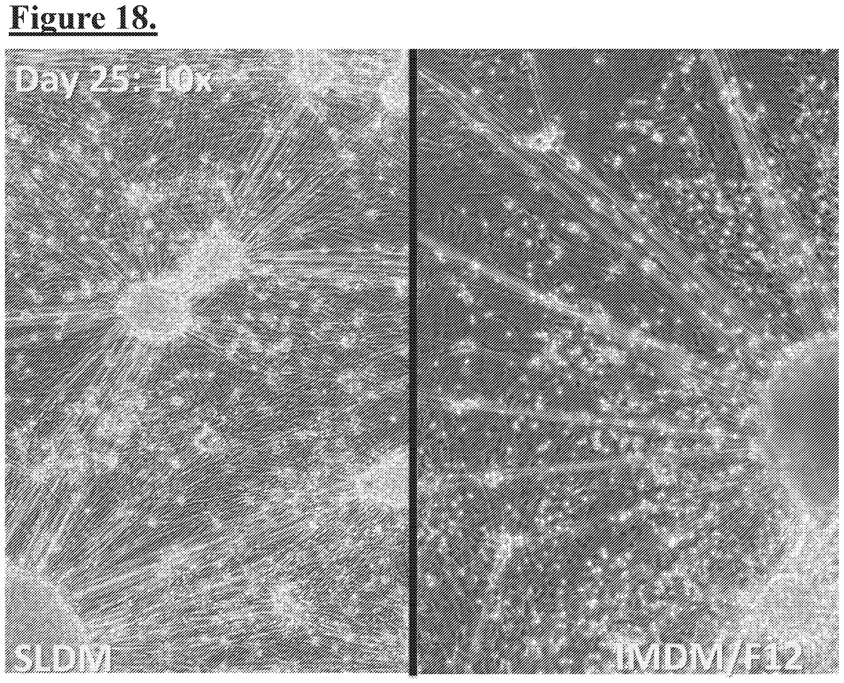

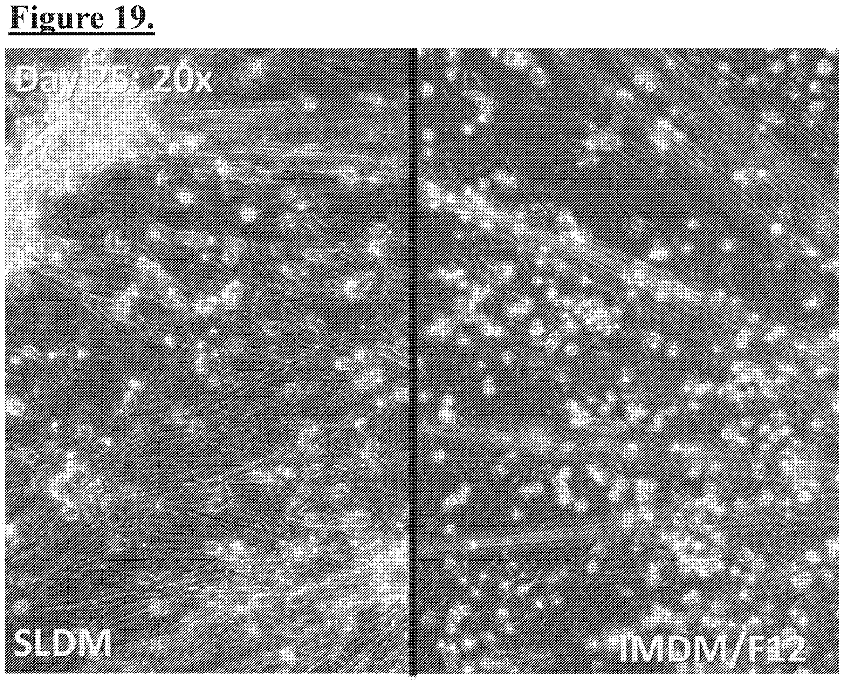

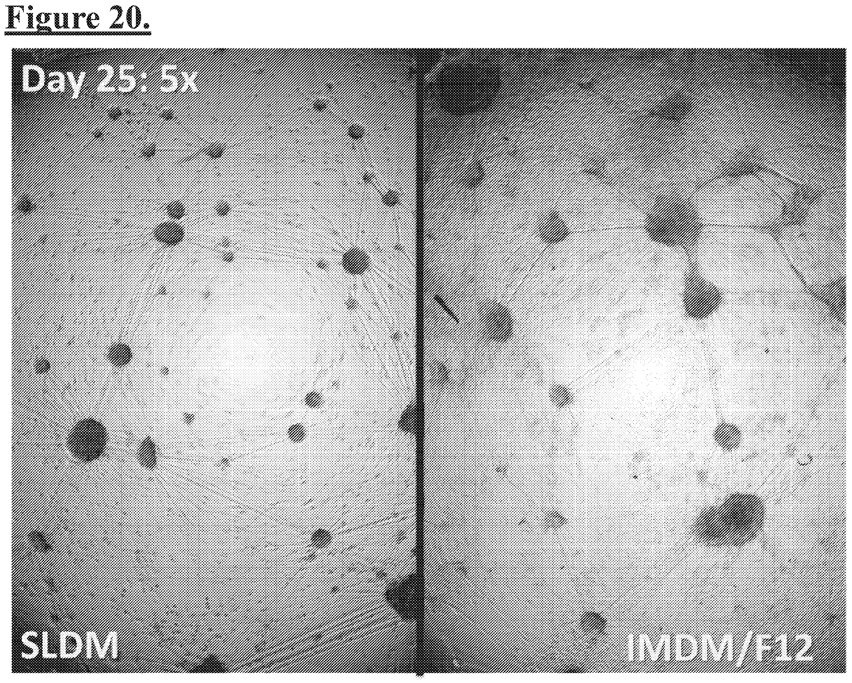

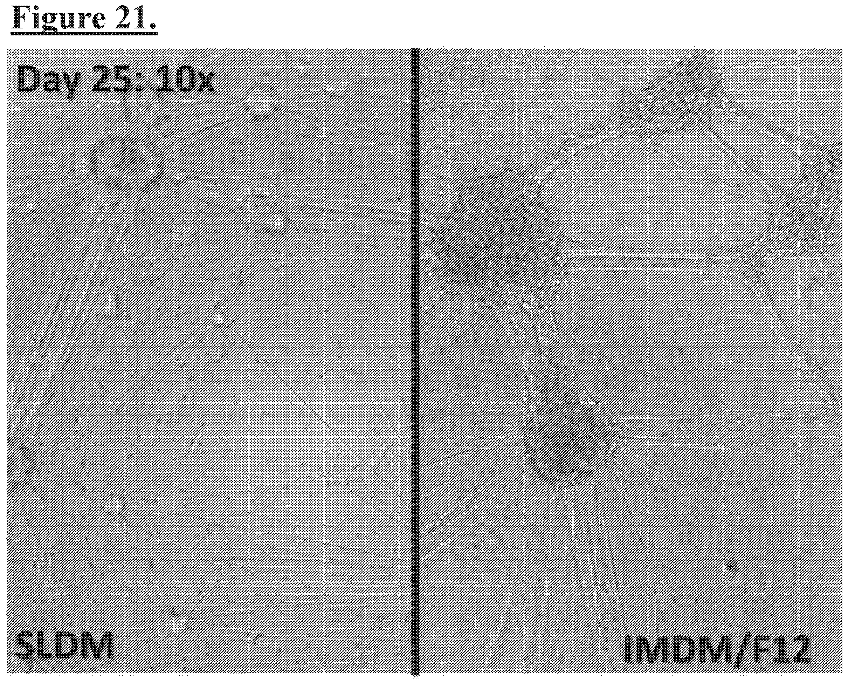

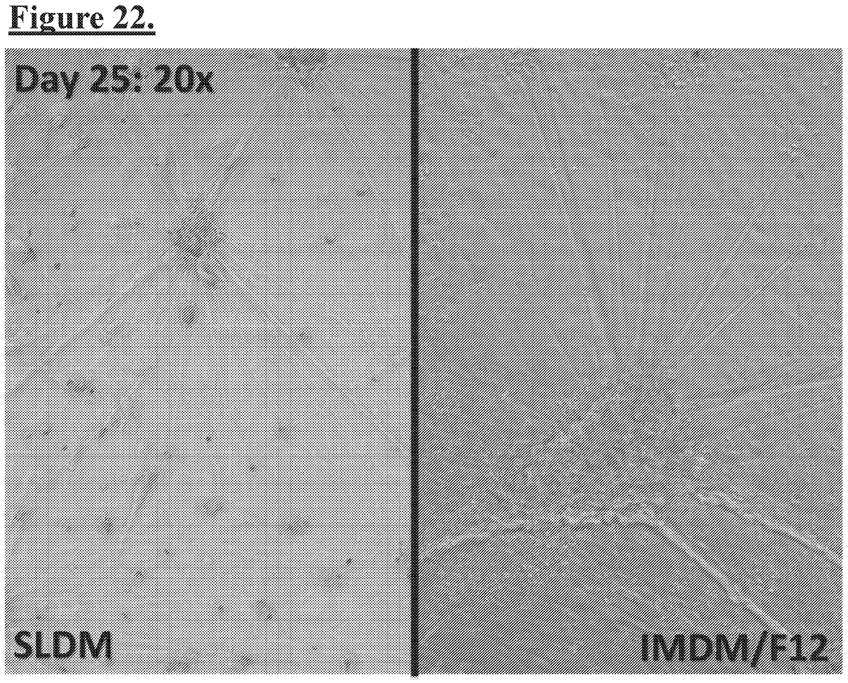

The Inventors developed an improved serum-free reformulation of B27. The Inventors found that B27 included high levels of proteins like catalase and superoxide dismutase, which may confound disease phenotypes related to oxidative stress and mitochondria. For example, neurons differentiated in the Inventors' SLDM vs IMDM media from C9orf72 mutant ALS patient neurons made higher level of dipeptide repeats (disease phenotype of mutant C9orf72 ALS neurons) in the SLdiMN media. Utilizing the SLDM media formulation, the Inventors were able to achieve reliable differentiation of spinal motor neurons across multiple different donor iPSCs, with improved plating and seeding of cells at day 6 (minimal # of fails). *See* Figs. 1-22. The SLDM media with supplements can fully serve as a replacement to IMDM/F12, and B27.

The following instructions will make 200 mL of 50x supplement. Each 10 mL aliquot can supplement 500 mL of differentiation medium.

**Table 1. Supplement Concentrate**

| **Reagent** | **[Stock]** | **[Supplement]** | **200 mL** |
|---|---|---|---|
| DMEM/F12 | 1x | 1x | Up to 200 mL |
| Albumin, Bovine Serum | Powder | 125 mg/mL | 25 g |
| Sodium Bicarbonate | Powder | 27.15 mg/mL | 5.43 g |
| L-Ascorbic Acid | Powder | 3.2 mg/mL | 640 mg |
| Putrescine | Powder | 805 ug/mL | 161 mg |
| D(+)-Galactose | Powder | 750 ug/mL | 150 mg |
| Holo-transferrin | Powder | 250 ug/mL | 50 mg |
| Catalase | Powder | 125 ug/mL | 25 mg |
| L-Carnitine | Powder | 125 ug/mL | 20 mg |
| Glutathione (reduced) | Powder | 50 ug/mL | 10 mg |
| | | | |
| Sodium Selenite | 1 mg/mL | 0.7 ug/mL | 140 uL |
| Ethanolamine | 1 g/mL | 50 ug/mL | 10 uL |
| T3 (triiodo-L-thyronine) | 2 mg/mL | 0.1 ug/mL | 10 uL |

| **Ethanolic Stocks** | | | |
|---|---|---|---|
| Corticosterone | 2 mg/mL | 1 ug/mL | 100 uL |
| Linoleic Acid | 100 mg/mL | 50 ug/mL | 100 uL |
| Linolenic Acid | 100 mg/mL | 50 ug/mL | 100 uL |
| Lipoic Acid (thioctic acid) | 4.7 mg/mL | 2.35 ug/mL | 100 uL |
| Progesterone | 3.2 mg/mL | 0.32 ug/mL | 20 uL |
| Retinol Acetate | 20 mg/mL | 5 ug/mL | 50 uL |
| a-Tocopherol (vitamin E) | 100 mg | 50 ug/mL | 100 uL |
| a-Tocopherol acetate | 100 mg | 50 ug/mL | 100 uL |

### Procedure:

1) Dissolve 25 g of Bovine Serum Albumin in 160 mL of DMEM/F12.
   Stirring is necessary and dissolving the solution on ice may be impossible. Perform at room temperature with the stir speed set as low as possible.
2) Dissolve 5.43 g of Sodium Bicarbonate in the solution.
   Weigh out and prepare the other components while the initial components are dissolving.
3) Dissolve 640 mg of L-Ascorbic acid in the solution.
4) Dissolve 161 mg of Putrescine.
5) Dissolve 150 mg of Galactose.
6) Add 50 mg of Holo-transferrin; 25 mg of Catalase; 20 mg of Carnitine; and 10 mg of Glutathione.
7) Combine 140 uL of Sodium Selenite; 10 uL of Ethanolamine; and 10 uL of T3 with 8 mL of DMEM/F12.
   Prepare this solution in a 15 mL conical tube while the powdered components are dissolving.
8) Add 100 uL each of Corticosterone, Linoleic Acid (LA), Linolenic Acid (LnA), Lipoic Acid (LpA), Tocopherol; and Tocopherol Acetate to the secondary solution.
9) Add 50 uL of Retinol Acetate and 40 uL of Progesterone to the secondary solution
10) Add the secondary solution to the primary solution.
11) Bring the total solution up to 200 mL
12) Let sit overnight at 4 C.
13) Gently stir, then aliquot twenty aliquots of 10 mL each. Label and store at -20 C.

**Table 2. Sareen Lab Differentiation Media (SLDM) including supplement**

| **Reagent** | **[Stock]** | **Location** | **[Final]** | **500 mL** | |
|---|---|---|---|---|---|
| DMEM/F12 | 1x | 4° C, Gomez | - | 500. | mL |
| SLDM Supplement | 50x | -20° C, Wednesday | 1x | 10. | mL |
| PSA (Anti-anti) | 1x | 4° C, Gomez, or Fester | 1% | 5. | mL |
| Glutamax | 100x | 4° C, Gomez | 1x | 5. | mL |
| Superoxide Dismutase | 2.5 mg/mL | -20° C, Fester, SODM box | 2.5 □g/mL | 500. | □L |
| Insulin | 10 mg/mL | 4° C, Gomez | 4 ug/mL | 200. | □L |

### Procedure:

1) Add SLDM supplement to DMEM/F12 in a stericup
2) Add 5 mL of PSA
3) Add 5 mL of Glutamax
4) Add 543 uL of Superoxide dismutase
5) Add 200 uL of Insulin
6) Mix and vacuum filter
7) Store at 4 C covered in aluminum foil

Using SLDM media and supplement, the Inventors observed higher survivability of cells after dissociation and re-plating. This media further allowed for cryo-preservatio and reliable thawing at day 12. The cryopreservation, using CryoStore CS10, capability allows for banking of large number of spinal cord progenitor cells for screening and regenerative applications in the future.

Differentiating spinal cord neurons can be maintained in this media up to 90 days, with the ability to manipulate and modify individual components that are packaged into commercially and widely used B27/N2.

### Example 3

### Chip microfabrication and culture

The Chip was fabricated by using modified methods for Chip microfabrication as previously described. Briefly, PDMS pre-polymer was mixed at a 10:1 ratio of PDMS base to curing agent, wt/wt using a planetary mixer (Thinky ARE-310). PDMS pre-polymer was then cast onto molds forming the microchannels of the upper layer (1,000 µm wide x 1,000µm high) and lower layer (1,000µm wide × 200µm high). The membrane was cast onto a silicon mold that was fabricated using photolithography and deep reactive ion etching, resulting in 7um pores. The components were cured overnight and removed from the mold. The upper layer, membrane, and lower layer were permanently bonded via plasma bonding to form the complete Chip. Chips then were treated with plasma in 100% oxygen for 2:00 minutes and immediately coated with Matrigel for the neural channel and a mixture of collagen IV (Invitrogen), Fibronectin (Invitrogen), and diluted in water in a ratio of 1:4:5 for the vascular channel. Coated chips were then incubated overnight at 37°C and 5% CO₂. Chips were seeded with BMECs at a concentration of 25,000 cells/µL and allowed to attach to the membrane by turning the chip over and incubating for 3 hours. Later spNPCs were thawed and seeded at a density of 6000 cells/µL and incubated overnight before flushing with fresh media. Media was then replaced every other day with approximately 25µL in main channel and an extra 25µ in reservoir tips on either side.

### Example 4

### Immunohistochemistry

Fetal tissue was received from the Birth Defects Research Laboratory at the University of Washington under their approved Institutional review board (IRB), consent and privacy guidelines. Protocols were performed in accordance with the Institutional Review Board's guidelines at the Cedars-Sinai Medical Center under the auspice IRB-SCRO Protocol Pro00021505.

Spinal cord samples arrived with estimated age and as partially intact spinal columns which were partitioned into approximated cervical, thoracic and lumbar sections. Fetal tissue was subsequently fixed in 4% paraformaldehyde for 48 hr, and placed in 30% sucrose for an additional 24 hour. Finally, spinal cords were embedded in Tissue-Tek OCT (VWR) and sectioned at 25 µm using a cryostat (Leica) at -20 °C and directly mounted on glass slides (Fisher Scientific). Tissue sections were permeabilized in cold MeOH for 20 minutes, and blocked in PBS containing 5% normal donkey serum (Sigma, D9663) and 0.25% Triton-X for 1.5 hour, then transferred to primary antibody solution containing mouse anti-GLUT1 (R&D Systems, 1:100) SMI-32P-100 (Covance, 1:1,000), and goat anti-Islet-1 (R&D, AF1387, 1:500), and rabbit anti-NFH (Sigma, N4142, 1:1000), rabbit anti-SIRT (Sigma, SAB4502861, 1:100) and incubated overnight at 4 °C. Samples were then incubated for 1 h at room temperature in donkey anti-mouse Alexa Fluor 488 and donkey anti-goat 594 secondary antibodies (Life Technologies, A21202 and A21289, 1:1,000 each). Fetal samples were mounted in Fluoromount-G (Southern Biotech, 0100-01) and acquired at 20x using automated stitching on a Leica DM 6000 microscope for whole mount image.

iPSC-derived cultures were fixed in 4% paraformaldehyde, and rinsed with PBS. Cultures were permeabilized in 10% Triton X at RT for 10 min and blocked in 5% donkey serum and 0.1% Triton-X at RT for 1 h. Samples were incubated overnight at 4 °C in primary antibody solution containing the following antibodies: mouse anti-SMI-32 (Covance, SMI-32P-100, 1:1,000), rabbit anti-ZO-1 (), mouse anti-GLUTl (GLUT1), mouse anti-Occludin (), mouse anti-Claudin5 (), rinsed in PBS, and incubated for 1 h at room temperature in donkey anti-mouse Alexa Fluor 488, donkey anti-goat 594, and donkey anti-goat 647 secondary antibodies (Life Technologies, A21202 and A21289, 1:1,000 each).

### Example 5

### Imaging and Population

Confocal images were acquired using an A1 confocal microscope (Nikon) using a Plan Apo 10x objective at 1-micron increments. Figure 24d was generated from auto-stitching three confocal stacks at 10x (Nikon Elements). Images were rendered with maximum projection and quantified using Imaris (Bitplane). For population and ISL1 nuclei size analysis, images were cropped to only top channel and max projected using IMARIS software. Sites were prioritized as containing maximal GFP positive nuclei in the frame. GFP positive nuclei were quantified using spots algorithm and ISL1 positive nuclei were quantified by filtering by GFP co-localization.

### Example 6

### Transcriptomic analysis

BMECs were seeded in endothelial cell media into either a T75 flask, or the bottom channel of the SC-chip. SNPs were thawed and seeded into either the 96 well plates or the top channel of the Chip and incubated overnight. The following day, media was replaced with Stage 3 media in all conditions. BMEC flask was washed 2x with minimal neural media (IMDM:F12 0.5% N2, 1% B27, 0.5x NEAA, 1x PSA). 24 hours later BMEC media was collected, centrifuged and filtered. BMEC conditioned media was then supplemented with remaining ingredients for Stage 3 media. Media for all conditions was replaced every two days. At 6 days post-seeding, samples were dissociated with Accutase (Sigma-Aldrich). 3 Chip or 3 wells of a 96 well plate were pooled for each experimental replicate for a total of three replicates. Pooled samples were washed in PBS, resuspended in cold MACS buffer (Miltenyl Biotech), and filtered using a 40-micron screen. Cells were sorted using an Influx FACS sorter (BD). GFP positive gating was established using SC-chip seeded exclusively with 83iGFP MNs for a positive control, and 83iCTR BMECs for a negative control. Samples were sorted using this established gating. Positive fraction cell pellets were frozen at -80°C until processing. For population counts at day 30, both channels were dissociated as before, and one Chip per replicate was quantified by number of GFP positive events.

### Example 7

### Live calcium transient imaging and analysis

Fluo-4 calcium dye (Invitrogen), was prepared at 10 mM in 50% Pluronic solution and DMSO, and diluted to a final concentration of 20 µM in ECS. Tissue cultures were incubated at RT for 30 minutes, then washed in fresh ECS and incubated an additional 30 minutes before acquiring. After a 2-minute burn in phase, 16 bit videos were acquired for 3 minutes at 20Hz on an Eclipse Ti microscope (Nikon) using a Plan Flor 20x objective (Nikon) equipped with an Orca -Flash4.0LT digital camera (Hamamatsu). As no difference in event detection was determined between 16- and 8-bit data, all datasets were down-sampled to 8-bit (ImageJ). Calcium activity was counted by tracing ROI in blinded fashion, i.e. the treatment conditions were not associated with the results, and creating masks for use in extracting intensity data (ImageJ). 20-50 neurons were counted per site, and at least 3 Chip or wells were included for each condition. A total of 148-400 neurons per condition were analyzed using MATLAB. dF/F traces were extracted through FluoroSNNAP. Automated calcium event detection was accomplished through template libraries described previously at a threshold of 0.05. Events were then curated manually by a blinded counter, in other words a person evaluated the events without knowing the associated treatment conditions.

### Example 8

### Seeding chips using Day 12 Spinal neural progenitor (SNP) cells

SNPs (fresh or thawed) were seeded into the top channel of the SC-chip and incubated for 6 days. SNPS or MNs were cultured in Stage 3 media (after day 12) consisting of IMDM/F12 (Gibco), B27, N2, 1% NEAA, 200ng/ml ascorbic acid, 0.5 uM retinoic acid, 0.1 uM cAMP, 0.1 uM SAG, lOng/ml GDNF, 10 ng/ml BDNF, 1% PSA and fed every two days.

After 6 days, cultures underwent immunostaining of cells in the main channel of the chip, including but not limited to markers of spMNs SMI32, nuclear marker isletl (ISL1), Beta 3 tubulin (TUBB3), NKX6.1, neurofilament marker microtubule-associated protein 2 (MAP2), and synaptic marker synaptophysin (SYNP).

### Example 9

### Seeding chips using Day 12 Spinal neural progenitor (SNP) cells and BMECs in SC-Chip

Tall channel microphysiological systems (Emulate Inc.) were treated with plasma in 100% oxygen for 2:00 minutes and immediately coated with matrigel for the neural channel and a mixture of collagen IV (Invitrogen), Fibronectin (Invitrogen), and diluted in water in a ratio of 1:4:5 respectively, and incubated overnight at 37°C and 5% CO₂. Chips were seeded with MNs and BMECs on the same day sequentially by flipping the Chip and allowing cells to attach to the membrane by gravity. Chips were fed with approximately 25uL of Stage 3, with an extra 25uL in reservoir tips and media was replaced every other day.

### Example 10

### Neurons in Spinal Cord-Chip develop and are spontaneously active

To study the neurogenic consequences of Organ-Chip microenvironment, a rapid protocol was developed to derive first neural ectoderm ventralized spinal neural progenitor cells (spNPC) from healthy control human fibroblast-derived iPSCs in 12 days that could be frozen and banked for future experiments (Figure 23a). spNPCs were seeded into the top channel of the dual-channel Spinal Cord-Chip constructed of polydimethylsiloxane (PDMS) (Emulate Inc.) (Figure 23b). Top and bottom channels of the chip are separated by a 50-micron thick membrane perforated by 7-micron diameter pores spaced 40-microns apart from center to center. Within 6 days of incubation in the top channel, mixed neural cultures expressed markers of developing spinal motor neurons (spMN), including motor neuron progenitor markers NKX6.1 and TUBB3, and spMNs as indicated by co-expression of the markers islet-1 (ISL1) and phosphorylated neurofilament heavy chain (SMI32) (Figure 23c,d). Neural cultures also stained positive for MAP2 and synaptophysin, indicating that synaptogenesis was initiated within the Chip.

Spontaneous neuronal activity drives developmental programs and is essential for the formation of mature neuronal circuits in the central nervous system (CNS) (Moody and Bosma 2005). To determine if the Spinal Cord-Chip culture was amenable to spontaneous neuronal activity, cultures were treated with calcium-activated dye Fluo-4 and florescent activity was acquired at 20 Hz for 3 mins (Figure 23e). By plotting the change in fluorescence with respect to time (dF/f), neuron-specific calcium transient events were characterized by fast onset and slow decay, consistent with developing neurons *in vitro* (Figure 23f). Calcium transient event detection in the Spinal Cord-Chip showed extensive activity in the relatively early cultures, providing evidence of accelerated development of neural activity and connectivity (Figurelg).

### Example 11

### BMECs infiltrate into top channel of Spinal Cord-Chip and form a barrier.

To recreate vascular-neural interactions *in vitro,* BMECs from the same 83CTR iPSC line were derived using an established protocol and seeded into the bottom channel of the Spinal Cord-Chip (Figure 24a). After 6 days in culture, immunostaining with GLUT-1 and SMI32 revealed clear separation of the two cultures in the top (neural) and bottom (endothelial) channels along the entire Chip (Figure 24b-d). BMECs exhibited cobblestone morphology typical of endothelial culture *in vitro* and expressed endothelial tight junction proteins zona occludens 1 (ZO-1), glucose transporter 1 (GLUT-1), and Claudin 5 (Figure 24b-d, S1b). BMEC cultures also expressed nuclear ISL1, previously unreported in human vascular endothelium (Figure 24c). To confirm vascular ISL1 expression *in vivo,* human fetal spinal cord sections at post-conception days 53 and 67 were immunostained for ISL1 and GLUT-1 and found to be co-expressed within infiltrating vasculature (Figure 248). While SMI32-positive soma were not found in the bottom channel, ZO-1-positive SMI32-negative regions in the top channel revealed infiltration of BMECs (Figure 24c-d). Confocal 3D reconstruction of the main channel displayed clear distinction of the two cultures with confluent BMECs surrounding the entire bottom channel that formed a continuous vessel-like tube (Figure 24d).

The Inventors further discriminated the infiltration of both cell types in the Spinal Cord-Chip co-culture. The 83iCTR iPSC line was engineered to constitutively express green florescent protein (83iGFP) with a nuclear localization signal by inserting the cassette into the AAVS1 locus using a zinc finger nuclease. 83iGFP iPSCs were then differentiated into spNPCs and seeded into the top channel as before, while BMECs were generated from the wildtype iPSC line and seeded into the bottom channel (Figure 24e). This allowed for clear distinction of GFP-positive MN cultures from GFP negative, ISL1-positive expressing BMECs. 83iGFP spNPCs seeded into the top channel without BMECs in the bottom channel readily infiltrated through the membrane pores into the bottom channel. In contrast, when 83iCTR-BMECs were included in the bottom channel, GFP-positive spNPC infiltration was effectively blocked (Figure 24e). In the top channel, defined regions of GFP negative/ISL1-positive nuclei were observed consistent with ZO-1 staining showing BMEC infiltration from the bottom channel (Figure 24c). To determine the influence of BMEC contact on spMN culture development, top channel GFP positive nuclei were quantified and assessed for ISL1 positive expression. There was no significant difference in GFP⁺ spMN nuclei in the presence of BMECs (Figure 24f-h). In addition, the percent ISL1-positive nuclei were not significantly different, indicating BMECs did not significantly affect MN differentiation.

### Example 12

### Chip and BMECs increase spontaneous activity of spinal neural cultures

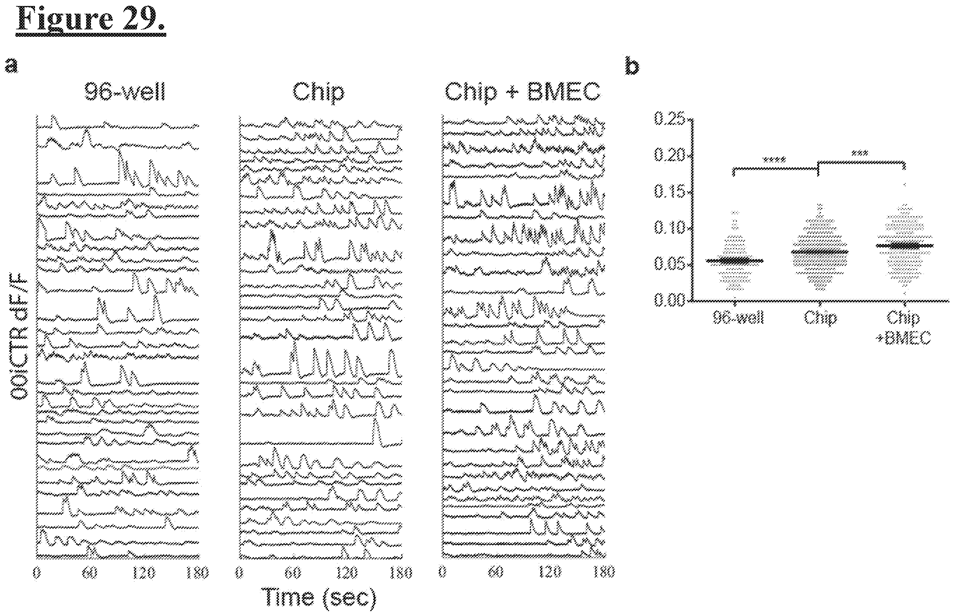

The Inventors next asked whether exposure of spNPC cultures to either the chip microenvironment and/or BMEC led to enhanced MN physiological function compared to culture in a 96-well (96W) plate. Live imaging and calcium-activated dye Fluo-4 were used to assess neuronal activity in these density-matched 83iCTR spNPC culture environments. Calcium transient signatures of BMECs analyzed in isolation at the seeding end were characterized by gradual influx and efflux that was distinct from the neural compartment (Figure 25a). To ensure that only neuron activity was analyzed in all conditions, putative BMEC activity was filtered out by requiring events to return to baseline within 20 seconds. Transient events for 148-300 neurons from each condition were plotted and quantified (Figure 25b). Results showed that there was a significant increase in the average number of spontaneous neuronal transients in the Spinal Cord-Chip cultures compared to 96-wells (Figure 25c). Of note, neurons cultured with BMECs in the Spinal Cord-Chip had a significantly increased frequency of calcium transients compared to culture in the Spinal Cord-Chip alone. Transient fluorescence was effectively blocked in the presence of voltage-gated sodium channel antagonist tetrodotoxin (TTX), indicating fluorescence specificity to channel activity. These findings were reproduced using an additional control iPSC line, 00iCTR (Figure 29a-b).

As iPSC-derived MN cultures commonly contain a mixture of both interneurons and excitatory MNs, the Inventors next wanted to determine if MNs themselves were specifically activated in the chips. To achieve population identification of calcium imaging data, cultures from the 00iCTR activity study were immunostained for motor neuron markers SMI32 and ISL1 and merged with the same site of live calcium acquisition (Figure 25d). 114 SMI32⁺/Isl1⁺ MNs were compared to 232 representative active neurons from the same acquisition site. ISL1⁺/SMI32⁺ MNs had significantly less activity when compared to overall neuronal activity (Figure 25e). Calcium events were detected in ISL1⁺/Smi32⁺ MNs for each culture environment (Figure 25f). Interestingly, increased activity in SMI32⁺/Isl1⁺ MNs was consistent with overall increased activity observed in chip cultures when compared 96-well plates (Figure 29e). Rare populations of MNs were highly active in the BMEC containing chips, however no overall increase in average transient frequency in MNs was observed compared to spNPCs cultured in Chip alone. This indicated that MNs within the mixed culture were more functional compared to the 96-well plate, and that MN activity with the addition of BMECs was more diverse.

### Example 13

### Chip microenvironment and BMEC co-culture induce distinct transcriptomic signatures

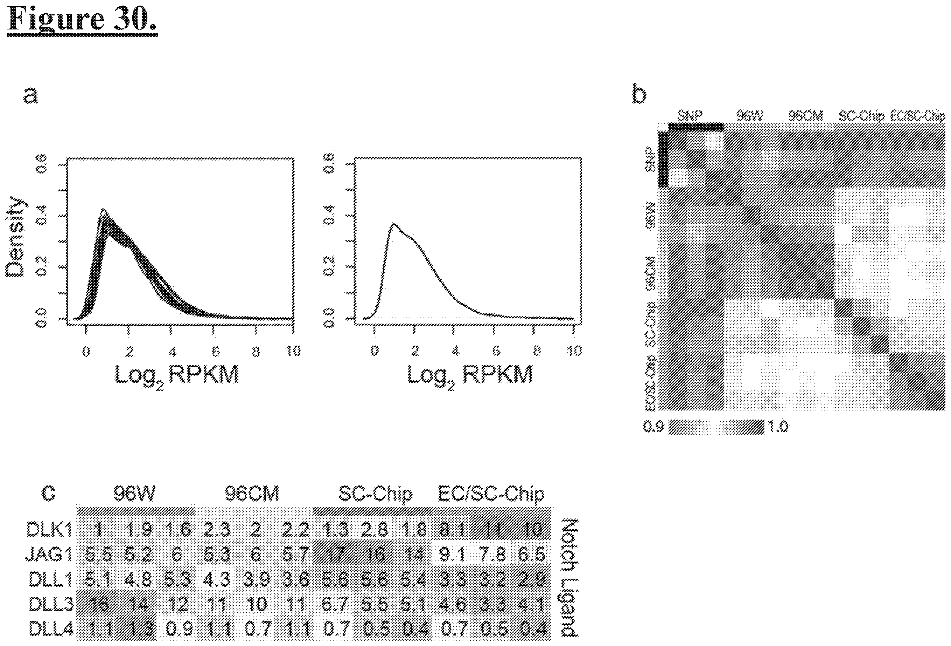

While calcium imaging is an important marker of neuronal activity, it does not capture certain aspects of neuronal maturation and cell-cell interaction. To address these, the Inventors performed RNA sequencing on spNPC cultures in the Spinal Cord-Chip alone or with BMEC co-culture. Day 12 GFP-positive spNPC cultures were thawed and then cultured for 6 days in 4 conditions: 96W, 96W with BMEC conditioned media (96CM), Spinal Cord-Chip seeded with spNPCs alone (Chip) or with the addition of BMECs (Chip+BMEC). After 6 days, they were dissociated and the GFP-positive population (neural) was separated from the GFP-negative population (BMEC) by florescence-assisted cell sorting (FACS) (Figure 26a-b). A lack of BMEC-specific marker expression in the FACS sorted samples chips containing BMECs indicated that BMECs were effectively removed through FACS (Figure 26c). Thawed spNPCs were also included to determine differences in gene expression resulting from the 6 days in culture. 10002 genes expressing at least 0.5 RPKM in at least one condition were included in the dataset and RPKM data was quantile normalized across all samples (Figure 30a). Pearson correlation analysis showed high reproducibility among sample replicates (Figure 30b).

To discover signaling pathways activated in each condition, principal component analysis (PCA) was performed on all samples, and the top 200 ranked genes from each analysis were then entered into the network-based pathway analysis platform DAVID (Figure 25d-e). The greatest variance (PC1) separated the day 12 spNPCs from day 18 MN stage in all other conditions (Figure 25d). Day 18 MNs had higher expression of gene pathways involved in neural differentiation, neurogenesis, and axonal growth (Figure 25c). The next greatest variance (PC2) separated chip culture conditions (Chip and Chip+BMEC) from the two 96-well cultures (96W and 96CM) and were driven by genes associated with neural differentiation pathways that were specifically up-regulated in the chip microenvironment. However, PC2 did not show a notable difference between the 96W and 96CM conditions suggesting only a small, and possibly antagonistic effect of conditioned media on neural cultures grown in 96-well plates. Finally, PC3 was defined by genes that were differentially expressed when spNPCs were directly co-cultured with BMECs within the chips (Chip+BMEC). DAVID analysis highlighted genes associated with extracellular matrix, secreted signaling peptides, and endothelial cell interaction pathways.

### Example 14

### The chip microenvironment enhances neurogenic programs in spNPC cultures

Because PC2 elicited "Neural Differentiation" and "Response to Protein Stimulus" DAVID gene ontology categories, the Inventors further investigated Spinal Cord-Chip induced genes relating to neural maturation. Relative expression of genes contributing to both categories were compared by calculating a Z-score for each gene across all samples (Figure 26f,g). Genes associated with "Response to Protein Stimulus" were upregulated in both Spinal Cord-Chip and EC/Spinal Cord-Chips, suggesting increased endogenous signaling activity resulting from chip microenvironment. Among the induced genes, early growth response gene 1 (EGR1) is known to be rapidly transcribed in response to environmental stimulation and neural activation. Cyclin D1 (CCND1) has been shown to promote MN neurogenesis in the developing spinal cord in a cell cycle-independent manner, acting trough a Notch-signaling effector pathway (Lukaszewicz and Anderson 2011). Within the "Neural Differentiation" category, transcription factors involved in spinal cord fate specification were also induced (Figure 26g). PAX6 is an intermediate neural progenitor differentiation marker that regulates neuronal cell cycle exit in the spinal cord. GLI2 and GLI3 were also induced and are act to regulate dorsal-ventral patterning in the spinal cord. Additionally, Up-regulation of ISL1 expression (Figure 26g) confirmed staining in Spinal Cord-Chips and further supported increased differentiation resulting from the chip microenvironment.

### Example 15

### BMECs induce angioneurin and extracellular matrix gene expression in developing neural cultures in Spinal Cord-Chip

Angioneurin signaling pathways related to VEGF, notch and axon guidance mechanisms are known to play heavily on both neuronal and vascular maturation *in vivo.* These pathways were therefore probed to determine if BMEC activated these pathways when co-cultured with neurons in the Spinal Cord-Chip. Vascular endothelial growth factor (VEGF) is a major contributor to sprouting angiogenesis in response to hypoxia and has been implicated in neural maturation. VEGFA receptor, KDR was induced in response to BMEC contact (Figure 26h). The Notch signaling pathway is heavily implicated in both angiogenesis and neural patterning during development *in vivo.* Inhibition of the Notch pathway through small molecule γ-secretase inhibitors have been used to enhance iPSC-derived MN differentiation *in vitro* leading to further investigation into Notch pathway activity. (Figure 30c). Significantly increased expression of DLK1, a potent inhibitor of notch signaling, and down regulation of Notch activating ligands JAG1, DLL1, DLL3, and DLL4 was observed in response to BMEC co-culture in the Spinal Cord-Chip.

Within the "Vascular Development" category, cell surface proteins important for both vascular infiltration and neuronal outgrowth were expressed specifically in response to BMEC co-culture. Thrombospondin domain-containing Semaphorins 3A and 5A transcripts increased 2 and 5-fold respectively compared to Spinal Cord-Chip. Semaphorins have opposing effects on angiogenesis *in vivo,* and in neurons are required for axon guidance, synaptogenesis, and neural maturation. Chemokine receptor (CXCR4) and ephrin B2 (EFNB2) are cell surface guidance molecules that are both required for motor neuron axon outgrowth.

Extracellular matrix proteins were among the most significantly induced in response to BMEC interaction and contained proteins that are implicated in neural maturation. Thrombospondin 1 (THBS1) of astrocyte origin has been shown to facilitate synaptogenesis and maturation derived of iPSC-derived neurons when co-cultured *in vitro.* THBS1 is cleaved by A disintegrin and metalloproteinase with thrombospondin moteifs 1 (ADAMTS1), which was also significantly induced. While ADAMTS18 activity in the neurodevelopmental context is unknown, ADAMTS1 is expressed in motor neurons during spinal cord injury. Extracellular matrix component Tenacin-C (TNC) was also significantly induced and has been shown to mediate motor neuron axon outgrowth in zebrafish and avian models. Collagen is an important constituent of vascular basement membrane and neurovascular niche. Expression of collagen family members COL5A2, COL12A1, and most significantly COL14A1 (over 10-fold expression) were significantly expressed by neural cultures in response to BMEC inclusion into the Spinal Cord-Chip.

### Example 16

### Spinal Cord-Chip induces genes that are associated with fetal development

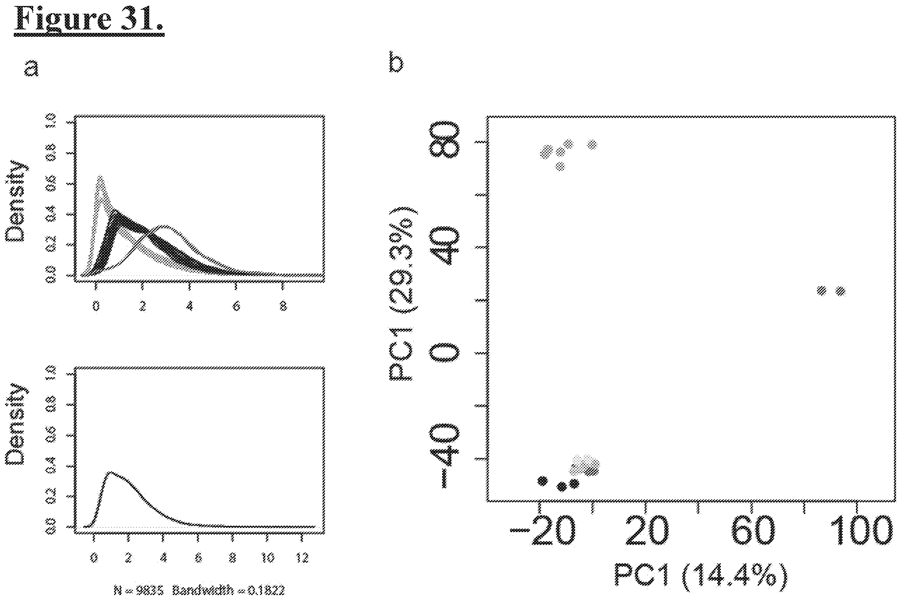

The pathways induced in response to BMEC co-culture in the Spinal Cord-Chip are known to be present in the developing CNS, however, cited studies were primarily generated using animal models. To ensure that induced pathways reflected those in the human developing spinal cord and to determine the extent to which these activated genes contributed to *in vivo* maturation, whole transcript data from the culture conditions were compared to datasets generated from primary human spinal cord tissue. Published transcriptomic profiles of fetal spinal cord (purple) and adult laser captured spinal motor neurons of a non-diseased cohort (green) were included and normalized to the 10002 genes that were analyzed in the culture experiments (Figure 31a). PCA performed on the 10,002 genes analyzed in the culture experiments (Figure 30b) illustrated a minor advancement towards *in vivo* fetal spinal tissue development and no differential effects among culture conditions. However, a more focused PCA based on the top and bottom 200 ranked gene loadings from PC2 and PC3 distinguished the culture conditions (Figure 25d). Dev-PC1 distinguished iPSC-derived neural cultures, fetal and adult samples in a similar fashion to the previous experiment (Figure 27a), and Dev-PC2 positively weighted genes corresponded to expression that reflected the human fetal spinal cord. In this reduced dataset, iPSC-derived conditions separated into discrete clusters and the Spinal Cord-Chip (blue) and EC/Spinal Cord-Chip (orange) conditions registered more positively along the Dev-PC2 axis and closer to the fetal samples, indicating that this condition induces gene expression changes relevant to and promoting *in vivo* spinal tissue development.

### Example 17

### The Spinal Cord Chip environment induces somatostatin expression in iPSC-derived spMNs

Dev-PC2 provided a ranked list of genes that contributed to the *in vivo* fetal signature along the most positively weighed genes. The Inventors therefore, sought to determine specific genes induced by Spinal Cord-Chip culture that promoted an *in vivo*-like spinal cord signature. The 50 highest-weighted Dev-PC2 genes were compared across all iPSC-derived cultures as before (Figure 27b). *Id* proteins (Id2/Id3) induced in the Spinal Cord-Chips were also highly expressed in the Fetal-SC samples. Angioneurin gene SEMA5A approached similar levels to *in vivo* samples, however, expression could not be confirmed by antibody staining (data not shown). The 6^{th} ranking gene in PC2 Somatostatin (SST) was highly expressed in the fetal Spinal Cord samples. In the brain *in vitro* and *in vivo,* SST has been shown to have trophic factor activity, influence synaptogenesis and axonal pathfinding and regulate cortical circuit maturation. Compared to 96W samples, Spinal Cord-Chip and EC/Spinal Cord-Chip environment induced 9 and 15-fold SST expression, respectively. To confirm protein expression *in vitro,* 96W and EC/Spinal Cord-Chips were cultured as before and stained with SST antibody along with MN marker ISL1 and SMI32. Pronounced SST staining was observed in the Spinal Cord-Chip both in the absence of BMECs, and to a greater extent with BMEC co-culture (Figure 27d). Expression was co-localized with SMI32 positive staining and proximal to ISL1 nuclear staining in the mixed culture, providing evidence that SST expression was specific to iPSC-derived spMNs. To determine if MN localization of SST was consistent with developing spMN pools *in vivo,* a day 67 human fetal spinal cord section was immunostained for SST and MN markers ISL1 and SMI32 as before (Figure 27e). ISL1 positive spMN pools could be visualized in the ventral horn that again contained positive staining for SST, confirming MN specificity *in vivo.*

### Example 18

### Discussion

In addition to BMEC's appreciated purpose as the gatekeepers to the brain, this model demonstrates the functional effects of iPSC-derived endothelial cells on neural development *in vitro.* Attempts to study the interplay of vascular endothelium on developing neural tissue have been challenged by inherent limitations to traditional approaches to co-culture. Transwell type cultures have been useful in the study of soluble signaling between primary murine BMECs from the adult neurovascular niche which report pro-proliferative effects through media conditioning that enhance neurogenic potential of primary cortical neural stem. However, soluble factors secreted by BMECs were not a major driver of proliferation, transcript expression, and spontaneous neural function in developing iPSC-derived cultures within the Chips. Whether this difference in soluble signaling is a result of iPSC origin and differentiation, or whether BMECs in the adult neurovascular stem cell niche are functionally distinct from the developing brain microvasculature context, or whether the difference is due to the specific advantage offered by the Organ-Chip system remains unclear. Prominent expression of Isl1 in both BMECs and MNs were observed in human spinal cord and iPSC-derived spMNs. Though BMEC ISL1 activity on vascular development and interaction with the developing neural tube is unknown, ISL1 knockout mice with ablated MN pools also displayed compromised vasculature indicating ISL1 requirement in vascular and MN development.

The advantages of a more functional system generated from including more of the cellular diversity of the spinal cord will likely increase physiological relevance further. Astrocytes are a critical component of the blood brain barrier and have been shown to increase neuronal maturation in co-culture. In addition, microglia colonization of the spinal cord begins at 9 weeks in human development is critical for synaptic pruning during development, and is implicated in neurodegenerative disease pathology such as C9Orf72 mutant ALS. Methods to derive astrocytes and microglia from human iPSCs have been described and could offer additional parameters to determine cell type contribution to and protection from disease. The multi-channel design of the chip system may allow for additional cell types to be seeded in a controlled manner to achieve an increased level of *in vivo*-like physiology.

The instability of cell attachment in PDMS chips remain a significant challenge for long term culture that if resolved could allow for additional maturation of iPSC-derived neural tissues. The hydrophobic nature of PDMS material represents unique challenges that could be mediated by altering surface properties through chemical treatments and refined ECM substrates. Additionally, the 7uM size of the membrane pores was ideal for interaction of BMECs to neural cells, however reduced diameters may be necessary to combat pronounced BMEC infiltration into the top channel for application of this system to test BBB penetrance of therapeutics into neural tissue. Constitutive media flow has been shown to be required for proper organ development in other tissues and could represent a novel parameter for accelerated development.

The function of SST in human developing spinal cord MNs remains largely unknown. In a separate study based on meta-analysis of published microarray data, SST was determined to be a major contributor to spinal cord maturity. This suggests SST could be a critical neuropeptide in human motor neuron development. Somatostatin receptor family member 1 is abundantly expressed in the mouse spinal cord and could mediate SST action, however intracellular SST as observed by immunostaining could also have non-canonical effects on MN development. The expression of SST as a result of Spinal Cord-Chip culture and in response to co-culture with BMECs indicates that the chip microenviroment and paradigm for co-culture may help increase physiological relevance of iPSC-derived neurological models which show great promise in elucidating key functional and pathological mechanisms of the human CNS.

The invention is defined in the appended claims.

## Claims

1. A method of generating spinal neural progenitor cells (spNPCs) comprising:
providing induced pluripotent stem cells (iPSCs);
differentiating iPSCs into neural ectodermal cells by culturing in neural induction media comprising LDN193189, SB431542, and CHIR99021 for about 5-7 days; and
dissociating neural ectodermal cells, replating on a cell culture substrate and further culturing the replated neural ectodermal cells in differentiation media for about 5-7 days to generate spNPCs,
wherein the differentiation media comprises retinoic acid and sonic hedgehog agonist (SAG).

2. The method of claim 1, wherein the iPSCs are cultured in neural induction media for a period of about 6 days.

3. The method of claim 1, wherein the replated neural ectodermal cells are cultured in differentiation media for a period of about 6 days.

4. The method of claim 1, wherein the differentiation media further comprises ascorbic acid.

5. A cell culture supplement, comprising:
Albumin, Bovine Serum, Sodium Bicarbonate, L-Ascorbic Acid, Putrescine, D(+)-Galactose, Holo-transferrin, Catalase, L-Carnitine, Glutathione (reduced), Sodium Selenite, Ethanolamine, and T3 (triiodo-L-thyronine), Corticosterone, Linoleic Acid, Linolenic Acid, Lipoic Acid (thioctic acid), Progesterone, Retinol Acetate, a-Tocopherol (vitamin E), and a-Tocopherol acetate.

6. A cell culture media comprising:
the cell culture supplement of claim 5, and a base media, optionally wherein the base media comprises DMEM/F12.

7. A method of generating spinal neural progenitor cells (spNPCs) comprising:
providing induced pluripotent stem cells (iPSCs);
differentiating iPSCs into neural ectodermal cells by culturing in neural induction media comprising LDN193189, SB431542, and CHIR99021 for about 6 days; and
dissociating neural ectodermal cells, replating on a cell culture substrate and further culturing the replated neural ectodermal cells in differentiation media for about 6 days to generate spNPCs,
wherein the neural induction media, differentiation media, or both, comprise a supplement comprising:
one or more first additives selected from the group consisting of: Albumin, Bovine Serum, Sodium Bicarbonate, L-Ascorbic Acid, Putrescine, D(+)-Galactose, Holo-transferrin, Catalase, L-Carnitine, Glutathione (reduced), Sodium Selenite, Ethanolamine, and T3 (triiodo-L-thyronine); and
one or more second additives selected from the group consisting of:
Corticosterone, Linoleic Acid, Linolenic Acid, Lipoic Acid (thioctic acid), Progesterone, Retinol Acetate, a-Tocopherol (vitamin E), and a-Tocopherol acetate, and
wherein the differentiation media comprises retinoic acid and sonic hedgehog agonist (SAG).

8. The method of claim 7, wherein the supplement comprises:
Albumin, Bovine Serum, Sodium Bicarbonate, L-Ascorbic Acid,
Putrescine, D(+)-Galactose, Holo-transferrin, Catalase, L-Carnitine, Glutathione (reduced), Sodium Selenite, Ethanolamine, T3 (triiodo-L-thyronine),
Corticosterone, Linoleic Acid, Linolenic Acid, Lipoic Acid (thioctic acid), Progesterone, Retinol Acetate, a-Tocopherol (vitamin E), and a-Tocopherol acetate.

9. The method of either claim 1 or claim 7, wherein the cell culture substrate is matrigel.

10. The method of either claim 1 or claim 7, wherein the differentiation media further comprises ascorbic acid.

11. A quantity of spNPCs made by the method of claim 7, wherein the spNPCs express NKX6.1, optionally TUBB3, and at least one of ISL1 and SMI32.

12. The method of either claim 1 or claim 7, further comprising freezing the spNPCs.

13. A cryopreserved solution of spNPCs made by the method of claim 12, wherein the spNPCs express NKX6.1, optionally TUBB3, and at least one of ISL1 and SMI32.

## Patentansprüche

1. Verfahren zum Erzeugen von spinalen neuralen Vorläuferzellen (spNPCs), umfassend:
Bereitstellen von induzierten pluripotenten Stammzellen (iPSCs);
Differenzieren von iPSCs in neurale ektodermale Zellen durch Kultivieren in neuralen Induktionsmedien, umfassend LDN193189, SB431542 und CHIR99021, für etwa 5-7 Tage; und
Dissoziieren neuraler ektodermaler Zellen, Replattieren auf einem Zellkultursubstrat und ferneres Kultivieren der replattierten neuralen ektodermalen Zellen in Differenzierungsmedien für etwa 5-7 Tage, um spNPCs zu erzeugen,
wobei das Differenzierungsmedium Retinsäure und Sonic-Hedgehog-Agonist (SAG) umfasst.

2. Verfahren nach Anspruch 1, wobei die iPSCs für eine Zeitdauer von etwa 6 Tagen in neuralen Induktionsmedien kultiviert werden.

3. Verfahren nach Anspruch 1, wobei die replattierten neuralen ektodermalen Zellen in Differenzierungsmedien für einen Zeitraum von etwa 6 Tagen kultiviert werden.

4. Verfahren nach Anspruch 1, wobei die Differenzierungsmedien ferner Ascorbinsäure umfassen.

5. Zellkulturzusatz, umfassend:
Albumin, Rinderserum, Natriumbicarbonat, L-Ascorbinsäure, Putrescin, D(+)-Galactose, Holotransferrin, Katalase, L-Carnitin, Glutathion (reduziert), Natriumselenit, Ethanolamin und T3 (Triiod-L-thyronin), Corticosteron, Linolsäure, Linolensäure, Liponsäure (Thioctsäure), Progesteron, Retinolacetat, a-Tocopherol (Vitamin E) und a-Tocopherolacetat.

6. Zellkulturmedium, umfassend:
den Zellkulturzusatz nach Anspruch 5 und ein Basismedium, wobei das Basismedium optional DMEM/F12 umfasst.

7. Verfahren zum Erzeugen von spinalen neuralen Vorläuferzellen (spNPCs), umfassend:
Bereitstellen von induzierten pluripotenten Stammzellen (iPSCs);
Differenzieren von iPSCs in neurale ektodermale Zellen durch Kultivieren in neuralen Induktionsmedien, umfassend LDN193189, SB431542 und CHIR99021, für etwa 6 Tage; und
Dissoziieren neuraler ektodermaler Zellen, Replattieren auf einem Zellkultursubstrat und ferneres Kultivieren der replattierten neuralen ektodermalen Zellen in Differenzierungsmedien für etwa 6 Tage, um spNPCs zu erzeugen,
wobei die neuralen Induktionsmedien, Differenzierungsmedien, oder beide, einen Zusatz umfassen, umfassend:
eines oder mehrere erste Additive, ausgewählt aus der Gruppe bestehend aus: Albumin, Rinderserum, Natriumbicarbonat, L-Ascorbinsäure, Putrescin, D(+)-Galactose, Holotransferrin, Katalase, L-Carnitin, Glutathion (reduziert), Natriumselenit, Ethanolamin und T3 (Triiod-L-thyronin); und
eines oder mehrere zweite Additive, ausgewählt aus der Gruppe bestehend aus: Corticosteron, Linolsäure, Linolensäure, Liponsäure (Thioctsäure), Progesteron, Retinolacetat, a-Tocopherol (Vitamin E) und a-Tocopherolacetat und
wobei das Differenzierungsmedium Retinsäure und Sonic-Hedgehog-Agonist (SAG) umfasst.

8. Verfahren nach Anspruch 7, wobei der Zusatz umfasst:
Albumin, Rinderserum, Natriumbicarbonat, L-Ascorbinsäure, Putrescin, D(+)-Galactose, Holotransferrin, Katalase, L-Carnitin, Glutathion (reduziert), Natriumselenit, Ethanolamin, T3 (Triiod-L-thyronin),
Corticosteron, Linolsäure, Linolensäure, Liponsäure (Thioctsäure), Progesteron, Retinolacetat, a-Tocopherol (Vitamin E) und a-Tocopherolacetat.

9. Verfahren nach entweder Anspruch 1 oder Anspruch 7, wobei das Zellkultursubstrat Matrigel ist.

10. Verfahren nach entweder Anspruch 1 oder Anspruch 7, wobei das Differenzierungsmedium ferner Ascorbinsäure umfasst.

11. Menge von spNPCs, hergestellt durch das Verfahren nach Anspruch 7, wobei die spNPCs NKX6.1, optional TUBB3, und zumindest eines von ISL1 und SMI32 exprimieren.

12. Verfahren nach entweder Anspruch 1 oder Anspruch 7, ferner umfassend Einfrieren der spNPCs.

13. Kryokonservierte Lösung von spNPCs, hergestellt durch das Verfahren nach Anspruch 12, wobei die spNPCs NKX6.1, optional TUBB3, und zumindest eines von ISL1 und SMI32 exprimieren.

## Revendications

1. Procédé de génération de cellules progénitrices neurales spinales (spNPC) comprenant :
la fourniture de cellules souches pluripotentes induites (iPSC) ;
la différenciation des iPSC en cellules ectodermiques neurales par culture dans un milieu d'induction neurale comprenant LDN193189, SB431542 et CHIR99021 pendant environ 5 à 7 jours ; et
la dissociation des cellules ectodermiques neurales, le ré-étalement sur un substrat de culture cellulaire et la culture supplémentaire des cellules ectodermiques neurales ré-étalées dans un milieu de différenciation pendant environ 5 à 7 jours pour générer des spNPC,
ledit milieu de différenciation comprenant de l'acide rétinoïque et un agoniste de Sonic Hedgehog (SAG).

2. Procédé selon la revendication 1, lesdites iPSC étant cultivées dans un milieu d'induction neurale pendant une période d'environ 6 jours.

3. Procédé selon la revendication 1, lesdites cellules ectodermiques neurales ré-étalées étant cultivées dans un milieu de différenciation pendant une période d'environ 6 jours.

4. Procédé selon la revendication 1, ledit milieu de différenciation comprenant en outre de l'acide ascorbique.

5. Complément pour culture cellulaire, comprenant :
de l'albumine, du sérum bovin, du bicarbonate de sodium, de l'acide L-ascorbique, de la putrescine, du D(+)-galactose, de l'holotransferrine, de la catalase, de la L-carnitine, du glutathion (réduit), du sélénite de sodium, de l'éthanolamine et de la T3 (triiodo-L-thyronine), de la corticostérone, de l'acide linoléique, de l'acide linolénique, de l'acide lipoïque (acide thioctique), de la progestérone, de l'acétate de rétinol, de l'a-tocophérol (vitamine E) et de l'acétate d'a-tocophérol.

6. Milieu de culture cellulaire comprenant :
le complément pour culture cellulaire selon la revendication 5, et
un milieu de base, éventuellement ledit milieu de base comprenant du DMEM/F12.

7. Procédé de génération de cellules progénitrices neurales spinales (spNPC) comprenant :
la fourniture de cellules souches pluripotentes induites (iPSC) ;
la différenciation des iPSC en cellules ectodermiques neurales par culture dans un milieu d'induction neurale comprenant LDN193189, SB431542 et CHIR99021 pendant environ 6 jours ; et
la dissociation des cellules ectodermiques neurales, le ré-étalement sur un substrat de culture cellulaire et la culture supplémentaire des cellules ectodermiques neurales ré-étalées dans un milieu de différenciation pendant environ 6 jours pour générer des spNPC,
ledit milieu d'induction neurale, ledit milieu de différenciation, ou les deux, comprenant un complément qui comprend :
un ou plusieurs premiers additifs choisis dans le groupe constitué par : l'albumine, le sérum bovin, le bicarbonate de sodium, l'acide L-ascorbique, la putrescine, le D(+)-galactose, l'holotransferrine, la catalase, la L-carnitine, le glutathion (réduit), le sélénite de sodium, l'éthanolamine et la T3 (triiodo-L-thyronine) ; et
un ou plusieurs seconds additifs choisis dans le groupe constitué par : la corticostérone, l'acide linoléique, l'acide linolénique, l'acide lipoïque (acide thioctique), la progestérone, l'acétate de rétinol, l'a-tocophérol (vitamine E) et l'acétate d'a-tocophérol, et
ledit milieu de différenciation comprenant de l'acide rétinoïque et un agoniste de Sonic Hedgehog (SAG).

8. Procédé selon la revendication 7, ledit complément comprenant :
de l'albumine, du sérum bovin, du bicarbonate de sodium, de l'acide L-ascorbique, de la putrescine, du D(+)-galactose, de l'holotransferrine, de la catalase, de la L-carnitine, du glutathion (réduit), du sélénite de sodium, de l'éthanolamine, de la T3 (triiodo-L-thyronine), de la corticostérone, de l'acide linoléique, de l'acide linolénique, de l'acide lipoïque (acide thioctique), de la progestérone, de l'acétate de rétinol, de l'a-tocophérol (vitamine E) et de l'acétate d'a-tocophérol.

9. Procédé selon la revendication 1 ou la revendication 7, ledit substrat de culture cellulaire étant le matrigel.

10. Procédé selon la revendication 1 ou la revendication 7, ledit milieu de différenciation comprenant en outre de l'acide ascorbique.

11. Quantité de spNPC préparée par le procédé selon la revendication 7, lesdites spNPC exprimant NKX6.1, éventuellement TUBB3, et au moins l'un parmi ISL1 et SMI32.

12. Procédé selon la revendication 1 ou la revendication 7, comprenant en outre la congélation des spNPC.

13. Solution cryoconservée de spNPC préparée par le procédé selon la revendication 12, lesdites spNPC exprimant NKX6.1, éventuellement TUBB3, et au moins l'un parmi ISL1 et SMI32.
